(19) Europäisches Patentamt
European Patent Office
Office européen des brevets

(11) EP 1 873 147 A1

(12) EUROPEAN PATENT APPLICATION
published in accordance with Art. 153(4) EPC

(43) Date of publication:
**02.01.2008 Bulletin 2008/01**

(21) Application number: **06732218.0**

(22) Date of filing: **21.04.2006**

(51) Int Cl.:
***C07D 237/32*** *(2006.01)*

(86) International application number:
**PCT/JP2006/308460**

(87) International publication number:
**WO 2006/115221 (02.11.2006 Gazette 2006/44)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.04.2005 JP 2005124247**

(71) Applicant: **Nippon Shinyaku Co., Ltd.**
**Kyoto-shi,**
**Kyoto 601-8550 (JP)**

(72) Inventors:
- **ZHANG, Xin**
  **Nagaokakyo-shi, Kyoto 6170826 (JP)**
- **KAGAYAMA, Kohei**
  **Yamashina-ku, Kyoto-shi,Kyoto 6078182 (JP)**

(74) Representative: **Vossius & Partner**
**Siebertstrasse 4**
**81675 München (DE)**

# (54) PHTHALAZINONE DERIVATIVE AND PHARMACEUTICAL COMPRISING THE SAME

(57) [Problem] The present invention is to provide a novel compound having an excellent PDE4 inhibitory activity and TNF-$\alpha$ production-suppressing activity, and also to provide a preventive and therapeutical agent for atopic dermatitis or the like.

[Means for Solution] The present invention includes a novel phthalazinone derivative of the following general structural formula [1] or a pharmaceutically acceptable salt thereof, and a pharmaceutical composition comprising it as an active ingredient.

[Chemical Formula 12]

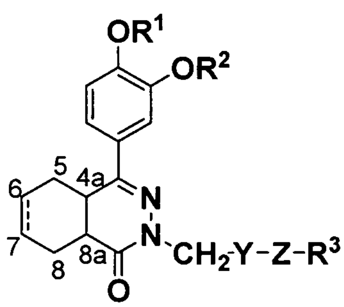

[1]

In the structural formula [1],
the following partial structure bridging the 6-position and the 7-position represents a single bond or a double bond:

[Chemical Formula 13]

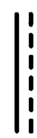

$R^1$ and $R^2$ are the same or different and each represents alkyl or the like, Y represents phenylene or the like, Z represents alkylene or the like, $R^3$ represents a mono- to tri-cyclic saturated or unsaturated cyclic amino group or the like, $R^{31}$ and $R^{32}$ represent alkyl or the like.

EP 1 873 147 A1

## Description

[Technical Field]

The present invention relates to a phthalazinone derivative and a pharmaceutical composition containing the derivative as an active ingredient.

[Background Art]

Important intracellular second messengers, cyclic adenosine 3',5'-monophosphate (hereinafter referred to as "cAMP") and cyclic guanosine 3',5'-monophosphate (hereinafter referred to as "cGMP") phosphorylate target proteins through activation of cAMP- and cGMP-dependent protein kinases, respectively, thereby modulating various cell functions (for example, see Non-Patent References 1 to 3). The regulation of intracellular concentration of cAMP and cGMP is well controlled by their production systems and degradation systems. Above all, their degradations are attained by hydrolases that are called phosphodiesterases (hereinafter referred to as "PDE") (for example, see Non-Patent References 4 and 5). Up to the present, at least 11 isozymes of PDE have been identified, referred to as type 1 PDE to type 11 PDE, and their characteristic structures, enzymochemical properties and tissue distribution and intracellular localization are known (for example, see Non-Patent References 6 to 8). Of those, type 4 PDE (hereinafter referred to as "PDE4") is a cAMP-specific PDE and is broadly distributed in tissues such as airway smooth muscle, liver, heart, brain and immune tissues.

PDE4 distributed in brain is a main PDE isozyme in the central nervous system of mammals. This isozyme significantly participates in the control of central nervous system function, for example regulating the cAMP concentration in neurocytes (for example, see Non-Patent References 81 to 84). Rolipram well known as a first-generation PDE4 inhibitor has a history that it was clinically developed as an antidepressant at its first discovery (for example, see Non-Patent References 85 and 86).

In addition, PDE4 is localized in immune cells and inflammatory cells that participate in inflammatory processes, such as lymphocytes, monocytes-/macrophages, mast cells, eosinophils, basophils, neutrophils. In these cells, PDE4 regulates the intracellular cAMP concentration, thereby controlling the functions of inflammatory cells, for example, activation of inflammatory cells and infiltration thereof into inflammatory sites, and activation of keratinocytes (for example, see Non-Patent References 9 to 12).

When PDE4 is inhibited, the cAMP concentration in these inflammatory cells may increase, whereby production and release of mediators such as histamine and leukotriene, and also production and release of various inflammatory cytokines such as tumor necrosis factor-$\alpha$ (hereinafter referred to as "TNF-$\alpha$") and interleukin (hereinafter referred to as "IL")-1, IL-2, IL-4, IL-5, IL-8 may be inhibited (for example, see Non-Patent References 13 to 22).

Of these inflammatory cytokines, TNF-$\alpha$ is a substance having an especially strong inflammation-inducing potency. TNF-$\alpha$ was first found as a soluble factor that has an effect of inducing necrosis of tumor cells (for example, see Non-Patent References 23 and 24). Afterwards, TNF-$\alpha$ has become interpreted as a strong inflammatory cytokine that not only causes systemic inflammatory reactions but also has a broad variety of physiological activities such as apoptosis induction, immunosuppression, cachexia, nonreversible shock, angiogenesis, and HIV virus replication (for example, see Non-Patent References 25 to 29).

Human TNF-$\alpha$ is produced as a membrane-bound protein of 26 kDa comprising 233 amino acids. After transportation onto the surface of a cell membrane, it is cleaved by TNF-converting enzyme to a soluble protein comprising 157 amino acids and having a molecular weight of 17 kDa, which is then released into extracellular milieu(for example, see Non-Patent References 30 and 31). TNF-$\alpha$ is produced mainly by activated macrophages during inflammation. When it acts on a TNF-$\alpha$ receptor expressed in immune cells, it activates transcription factors such as NF-$\kappa$B and AP-1, thereby inducing production of IL-1, IL-2, IL-6, IL-8 and so on and expression of IL-1 and IL-6 receptors. It is considered that, in a cytokine cascade to induce inflammatory reactions, TNF-$\alpha$ may control other inflammatory cytokines and their receptors at the uppermost stream. When TNF-$\alpha$ only is inhibited, production of IL-1, IL-6 and IL-8 may be inhibited. In this connection, it is known that inhibition of IL-1 alone could not inhibit the production of IL-6 and IL-8 (for example, see Non-Patent References 26, 32 to 38).

Further, it has been demonstrated that TNF-$\alpha$ has an effect of inducing expression of adhesion molecules such as E-selectin and chemokines such as monocyte chemoattractant protein-1 (MCP-1), and of enhancing the infiltration and accumulation of inflammatory cells from circulation into the local lesion sites such as inflamed skin, intestinal tracts and joints. In addition, TNF-$\alpha$ participates in inducing activation of proteases such as collagenases, C3 complement protein, NO synthetase, cyclooxygenase-2 (COX-2), etc., and also in producing various inflammatory mediators such as prostaglandin (for example, see Non-Patent References 39 to 43).

These facts suggest the possibility that a specific inhibition of a single molecule TNF-$\alpha$ could stop an inflammatory cytokine cascade reaction in its initial stage. Therefore, a broad-variety of inflammatory reactions could thereby be

inhibited pleiotropically. Accordingly, a TNF-α production inhibitor is expected to be a next-generation antiinflammatory agent having an extremely increased applicability to various inflammatory diseases, as compared with existing therapeutic agents (for example, see Non-Patent References 44 to 47).

Recently, it has been known that an anti-TNF-α antibody infliximab having a mechanism of TNF-α inhibition has a dramatic therapeutical effect for intractable chronic inflammatory diseases such as Crohn's disease, rheumatoid arthritis and psoriasis that are said to have close relation to TNF-α. Accordingly, in clinical practice, a TNF-α production inhibitor is categorized as an epoch-making new drug, and a therapeutic concept for chronic inflammation based on the TNF-α inhibitory effect has been established (for example, see Non-Patent References 48 to 50).

From the above, since a PDE4 inhibitor may inhibit production and release of a strong inflammation-inducing substance TNF-α as well as of various inflammatory cytokines such as IL-1, IL-2, IL-4, IL-5 and IL-8, it is expected to be able to inhibit the initiation, development and prolongation of inflammatory reactions propagated by these inflammation-inducing substances. Specifically, a PDE4 inhibitor is useful as a preventive/therapeutical agent for cytokine-related diseases, for example, many inflammatory or autoimmune diseases such as atopic dermatitis, contact dermatitis, psoriasis, allergic rhinitis, allergic conjunctivitis, chronic rheumatism, ulcerative colitis, Crohn's disease, sepsis, toxic shock syndrome, nephritis, hepatitis, circulatory insufficiency, multiple sclerosis, AIDS, allograft rejection, osteoarthritis, autoimmune diabetes, and autoimmune encephalomyelitis. In addition, it is expected to be a preventive/therapeutical agent for other diseases that are known to be correlated with PDE4, for example, asthma, chronic bronchitis, and chronic obstructive pulmonary disease (COPD) (for example, see Non-Patent References 51 to 61).

As mentioned above, a PDE4 inhibitor has an excellent, broad range of antiinflammatory effects but, on the other hand, it participates in the regulation of central nervous system function. Therefore, when a PDE4 inhibitor is used as a preventive/therapeutical agent for inflammatory or autoimmune diseases, its use may be limited owing to its side effects on central nervous system, such as nausea, emesis, and headache (see Non-Patent References 74, 76 to 80).

A compound having the following general structural formula [A] is known as a PDE4 inhibitor having phthalazinone skeleton(for example, see Patent Reference 1).

[Chemical Formula 3]

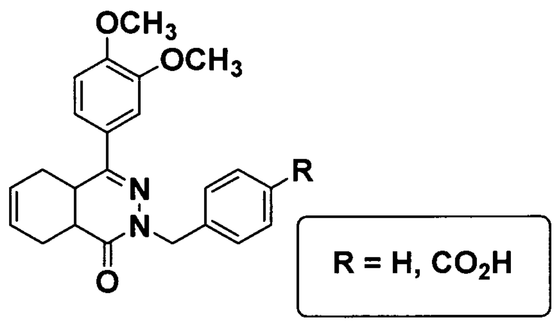

R = H, $CO_2H$

[A]

Patent Document 1: WO98/31674

Non-Patent Document 1: Biochem.Biophys.Acta., 1224, 467-479, 1994

Non-Patent Document 2: Trends Biochem.Sci., 22, 217-224, 1997

Non-Patent Document 3: Nat.Rev.Mol.Cell Biol., 3, 710-718, 2002

Non-Patent Document 4: Front Neuroendocrinol, 21, 103-132, 2000

Non-Patent Document 5: Curr.Opin.Cell Biol., 12, 174-179, 2000

Non-Patent Document 6: Mol.Pharmacol., 46, 399-405, 1994

Non-Patent Document 7: Drugs, 59,193-212,2000

Non-Patent Document 8: J.Bio.Chem., 278, 5493-5496, 2003

Non-Patent Document 9: Clin.Exp.Allergy, 22, 337-344, 1993

Non-Patent Document 10: Biochem.Pharmacol., 57. 965-973, 1999

Non-Patent Document 11: Eur.Respir.J., 8, 1179-83, 1995

Non-Patent Document 12: Pharmaco.Ther., 51, 13-34, 1991

Non-Patent Document 13: Am.J.Respir.Crit.Care Med., 157, 351-370, 1998

Non-Patent Document 14: JPET, 297, 267-279, 2001
Non-Patent Document 15: Biochem.J., 370, 1-18, 2003
Non-Patent Document 16: Br.J.Pharmacol., 123, 631-636, 1998
Non-Patent Document 17: Br.J.Pharmacol., 124, 547-555, 1998
Non-Patent Document 18: J.Allergy Clin.Immunol., 103, S421-S428, 1999
Non-Patent Document 19: Int.Arch.Allergy Immunol., 114, 348-353, 1997
Non-Patent Document 20: JPET, 278, 1356-1361, 1996
Non-Patent Document 21: Eur.J.Pharmacol., 367, 343-350, 1999
Non-Patent Document 22: J.Allergy Clin.Immunol., 99, 28-37, 1997
Non-Patent Document 23: PNAS, 72, 3666-3670, 1975
Non-Patent Document 24: Science, 230, 630-632, 1985
Non-Patent Document 25: Nat.Rev.Immunol., 3, 745-756, 2003
Non-Patent Document 26: Cell, 104, 487-501, 2001
Non-Patent Document 27: Science, 234, 470-474, 1986
Non-Patent Document 28: Chem.Immunol., 74, 141-161, 2000
Non-Patent Document 29: Eur.Cytokine Netw., 7, 93-124, 1996
Non-Patent Document 30: Nature, 385, 733-736, 1997
Non-Patent Document 31: Microsc.Res.Tech., 50, 184-195, 2000
Non-Patent Document 32: Lancet, 2,244-247, 1989
Non-Patent Document 33: Trends Cell Biol., 11, 372-377, 2001
Non-Patent Document 34: Nat.Cell Biol., 4, 131-136, 2002
Non-Patent Document 35: PNAS, 96, 3763-3768, 1999
Non-Patent Document 36: EMBO.J., 15, 1914-1923, 1996
Non-Patent Document 37: J.Bio.Chem., 273, 3285-3290, 1998
Non-Patent Document 38: Eur.Cytokine Netw., 6, 225-230, 1995
Non-Patent Document 39: Science, 285, 248-251, 1999
Non-Patent Document 40: J.Immunol., 159, 3508-3518, 1997
Non-Patent Document 41: Nature, 337, 661-663, 1989
Non-Patent Document 42: J.Bio.Chem., 268, 9526-9532, 1993
Non-Patent Document 43: J.Exp.Med., 162, 2163-2168, 1985
Non-Patent Document 44: Brit.J.Pharmaco., 135, 855-875, 2002
Non-Patent Document 45: Drug Discov.Today, 2, 273-282, 1997
Non-Patent Document 46: Annu.Rev.Immunol., 14, 397-440, 1996
Non-Patent Document 47: Annu.Rep.Med.Chem., 32, 241-250, 1997
Non-Patent Document 48: Lancet, 354, 1932-1939, 1999
Non-Patent Document 49: Gastroenterology, 109, 129-135, 1995
Non-Patent Document 50: N.Engl.J.Med., 340, 1398-1405, 1999
Non-Patent Document 51: J.Pharm.Pharmacol., 55(8), 1107-1114, 2003
Non-Patent Document 52: J.Invest.Dermatol., 107, 51-56, 1996
Non-Patent Document 53: Curr.Pharma.Design, 8, 1255-1296, 2002
Non-Patent Document 54: Annu.Rev.Med., 45, 491-503, 1994
Non-Patent Document 55: Curr.Opin.Cell Biol, 5, 432-438, 2001
Non-Patent Document 56: Trends Pharmacol.Sci., 18(5), 164-71, 1997
Non-Patent Document 57: Br.J.Pharmacol., 115, 39-46, 1995
Non-Patent Document 58: Nat.Rev.Drug Discov., 2, 736-746, 2003
Non-Patent Document 59: Arthritis.Res., 4, S17-524, 2002
Non-Patent Document 60: Drug News Perspect, 9, 261-269, 1996
Non-Patent Document 61: J.Inflamm., 46, 86-97, 1996
Non-Patent Document 62: J.Med.Chem., 44(16), 2511-2522, 2001
Non-Patent Document 63: J.Med.Chem.,44(16), 2523-2535, 2001
Non-Patent Document 64: J.Med.Chem.,45(12),2526-2533,2002
Non-Patent Document 65: Chem.Ber., 123, 523-533, 1990
Non-Patent Document 66: Chem.Ber., 123, 1885-1889, 1990
Non-Patent Document 67: Tetrahedron Lett., 41(32), 6241-6244
Non-Patent Document 68: J.Org.Chem., 63(17), 6023-6026, 1998
Non-Patent Document 70: J.Org.Chem., 66(23), 2001, 7729-7737
Non-Patent Document 71: J.Org.Chem., 65(4), 2000, 1144-1157
Non-Patent Document 72: Org.Lett., 5(14), 2003, 2453-2456

Non-Patent Document 73: Eur.J.Org.Chem., 1999, 9, 2373-2381
Non-Patent Document 74: Igakunoayumi, 2004, 210, 10, 883-887
Non-Patent Document 75: Org.Lett., 4(4), 2002, 581-584
Non-Patent Document 76: JPET., 287, 1998, 705-711
Non-Patent Document 77: Eur.J.Pharmacol., 286, 1995, 281-290
Non-Patent Document 78: Curr.Ther.Res., 38, 1985, 23-29
Non-Patent Document 79: Lancet., 358, 2001, 265-270
Non-Patent Document 80: Expert Opinion on Investigational Drugs, 10(4), 2001, 1361-1379
Non-Patent Document 81: Neuropsychopharmacology., 22, 2000, 42-51
Non-Patent Document 82: Prog.Nucleic Acid Res.Mol.Biol., 69, 2001, 249-315
Non-Patent Document 83: J.Neurosci., 19, 1999, 610-618
Non-Patent Document 84: Eur.J.Pharmaco., 498, 2004, 135-142
Non-Patent Document 85: Biochemica et Biophysica acta., 797, 1984, 354-362
Non-Patent Document 86: Neuropsychopharmacology., 22, 1983, 267-272
Non-Patent Document 87: Bioorg.Med.Chem.Lett., 2004, 14(21), 5275-5280
Non-Patent Document 88: J.Org.Chem., 2004, 1, 129-137

[Disclosure of the Invention]

[Problem to be Solved by the Invention]

A main object of the present invention is to provide a novel compound having an excellent PDE4-inhibitory activity and TNF-α production-suppressing activity. Further, the present invention is to provide a preventive and therapeutical agent for atopic dermatitis, contact dermatitis, psoriasis, allergic rhinitis, allergic conjunctivitis, chronic rheumatism, ulcerative colitis, Crohn's disease, sepsis, toxic shock syndrome, nephritis, hepatitis, circulatory insufficiency, multiple sclerosis, AIDS, allograft rejection, osteoarthritis, autoimmune diabetes, autoimmune encephalomyelitis, asthma, chronic bronchitis, and chronic obstructive pulmonary disease (COPD).

[Means to Solve the Problem]

The present inventors have earnestly studied and, as a result, have found that a novel phthalazinone derivative having the following general structural formula [1] (hereinafter referred to as the compound of the invention) or a pharmaceutically acceptable salt thereof attains the above object, and have completed the present invention.

[Chemical Formula 4]

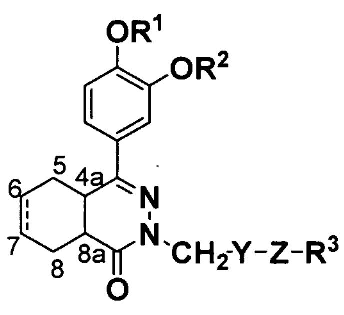

[1]

In the structure formula [1], the following partial structure bridging the 6-position and the 7-position represents a single bond or a double bond. That is, the structural formula [1] includes both a compound having a 4a,5,8,8a-tetrahydro-2H-phthalazin-1-one skeleton and a compound having a 4a,5,6,7,8,8a-hexahydro-2H-phthalazin-1-one skeleton. That partial structure in the structural formula shown in this description also has the same meaning.

[Chemical Formula 5]

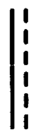

$R^1$ and $R^2$ are the same or different and each represents alkyl or cycloalkyl.
Y represents phenylene or a divalent aromatic heterocyclic group which is made up of 6 ring-constituting atoms and has 1 or 2 nitrogen atoms as the ring-constituting atoms.
Z represents a single bond, alkylene, alkenylene or alkynylene.
$R^3$ represents a mono- to tri-cyclic, saturated or unsaturated cyclic amino group which is made up of 5 to 10 ring-constituting atoms and has at least one nitrogen atom, and besides, optionally 1 to 3 nitrogen, oxygen or sulfur atoms as the ring-constituting atoms. The cyclic amino group may be substituted with $R^{31}$ and $R^{32}$, in which the nitrogen atom as the ring-constituting atom may form an oxide.
$R^{31}$ and $R^{32}$ are the same or different and each represents alkyl, alkoxy, hydroxy, halogen or oxo; or $R^{31}$ and $R^{32}$ are taken together to form methylenedioxy or ethylenedioxy.

The present invention also provides a pharmaceutical composition comprising a compound of the invention as an active ingredient; precisely, a preventive or therapeutical agent for atopic dermatitis, contact dermatitis, psoriasis, allergic rhinitis, allergic conjunctivitis, chronic rheumatism, ulcerative colitis, Crohn's disease, sepsis, toxic shock syndrome, nephritis, hepatitis, circulatory insufficiency, multiple sclerosis, AIDS, allograft rejection, osteoarthritis, autoimmune diabetes, autoimmune encephalomyelitis, asthma, chronic bronchitis, and chronic obstructive pulmonary disease (COPD).

In the above structural formula [1], the partial structure bridging the 6-position and the 7-position is preferably a double bond. That is, a compound having a 4a,5,8,8a-tetrahydro-2H-phthalazin-1-one skeleton is preferred.
$R^1$ and $R^2$ in the above structural formula [1] are the same or different and each is preferably alkyl.
Y in the above structural formula [1] is preferably phenylene.
Z in the above structural formula [1] is preferably a single.bond or alkylene, more preferably alkylene.
$R^3$ in the above structural formula [1] is preferably morpholin-4-yl, 4-oxido-morpholin-4-yl, piperidin-1-yl, piperazin-1-yl, thiomorpholin-4-yl or imidazol-1-yl optionally substituted with $R^{31}$ and $R^{32}$; and $R^{31}$ and $R^{32}$ are the same or different and each is preferably alkyl or oxo.

Specifically, the compound of the invention includes, for example, phthalazinone derivatives of any of the following (1) to (26) or their pharmaceutically acceptable salts.

(1) cis-4-(3,4-Dimethoxyphenyl)-2-[4-(morpholin-4-ylmethyl)benzyl]-4a,5,8,8a-tetrahydro-2H-phthalazin-1-one
(2) cis-(+)-4-(3,4-Dimethoxyphenyl)-2-[4-(morpholin-4-ylmethyl)benzyl]-4a,5,8,8a-tetrahydro-2H-phthalazin-1-one
(3) cis-(-)-4-(3,4-Dimethoxyphenyl)-2-[4-(morpholin-4-ylmethyl)benzyl]-4a,5,8,8a-tetrahydro-2H-phthalazin-1-one
(4) cis-4-(3,4-Dimethoxyphenyl)-2-[4-(piperidin-1-ylmethyl)benzyl]-4a,5,8,8a-tetrahydro-2H-phthalazin-1-one
(5) cis-4-(3,4-Dimethoxyphenyl)-2-[4-(1,4-dioxa-8-azaspiro[4,5]dec-8-ylmethyl)benzyl]-4a,5,8,8a-tetrahydro-2 H-phthalazin-1-one
(6) cis-4-(3,4-Dimethoxyphenyl)-2-[4-(4-oxopiperidin-1-ylmethyl)benzyl]-4a,5,8,8a-tetrahydro-2H-phthalazin-1-one
(7) cis-4-(3,4-Dimethoxyphenyl)-2-[4-(trans-2,6-dimethylmorpholin-4-ylmethyl)benzyl]-4a,5,8,8a-tetrahydro-2H-phthalazin-1-one
(8) cis-4-(3,4-Dimethoxyphenyl)-2-[4-(cis-2,6-dimethylmorpholin-4-ylmethyl)benzyl]-4a,5,8,8a-tetrahydro-2H-phthalazin-1-one
(9) cis-4-(3,4-Dimethoxyphenyl)-2-[4-(imidazol-1-ylmethyl)benzyl]-4a,5,8,8a-tetrahydro-2H-phthalazin-1-one
(10) cis-4-(3,4-Dimethoxyphenyl)-2-[3-(morpholin-4-ylmethyl)benzyl]-4a,5,8,8a-tetrahydro-2H-phthalazin-1-one
(11) cis-4-(3,4-Dimethoxyphenyl)-2-[2-(morpholin-4-ylmethyl)benzyl]-4a,5,8,8a-tetrahydro-2H-phthalazin-1-one
(12) cis-4-(3,4-Dimethoxyphenyl)-2-[6-(morpholin-4-ylmethyl)pyridin-2-ylmethyl]-4a,5,8,8a-tetrahydro-2H-phthalazin-1-one
(13) cis-4-(3,4-Dimethoxyphenyl)-2-[4-(morpholin-4-ylmethyl)benzyl]-4a,5,6,7,8,8a-hexahydro-2H-phthalazin-1-one
(14) cis-4-(3-Cyclopentyloxy-4-methoxyphenyl)-2-[4-(morpholin-4-ylmethyl)benzyl]-4a,5,8,8a-tetrahydro-2H-phthalazin-1-one
(15) cis-(+)-4-(3,4-Dimethoxyphenyl)-2-[4-(2-oxo-piperidin-1-ylmethyl)benzyl]-4a,5,8,8a-tetrahydro-2H-phthalazin-1-one
(16) cis-(+)-4-(3,4-Dimethoxyphenyl)-2-[4-(3-oxopiperazin-1-ylmethyl)benzyl]-4a,5,8,8a-tetrahydro-2H-phthalazin-1-one

(17) cis-(+)-4-(3,4-Dimethoxyphenyl)-2-[4-(1,1-dioxidothiomorpholin-4-ylmethyl)benzyl]-4a,5,8,8a-tetrahydro-2H-phthalazin-1-one

(18) cis-(+)-4-(3,4-Dimethoxyphenyl)-2-[4-(4-methyl-3-oxopiperazin-1-ylmethyl)benzyl]-4a,5,8,8a-tetrahydro-2H-phthalazin-1-one

(19) cis-(+)-4-(3,4-Dimethoxyphenyl)-2-[4-(2,6-dioxo-piperidin-1-ylmethyl)benzyl]-4a,5,8,8a-tetrahydro-2H-phthalazin-1-one

(20) cis-(+)-4-(3,4-Dimethoxyphenyl)-2-[3-(3-oxopiperazin-1-ylmethyl)benzyl]-4a,5,8,8a-tetrahydro-2H-phthalazin-1-one

(21) cis-(+)-4-{4-[4-(3,4-Dimethoxyphenyl)-1-oxo-4a,5,8,8a-tetrahydro-1H-phthalazin-2-ylmethyl]benzyl} morpholine 4-oxide

(22) cis-4-(4-Methoxy-3-propoxyphenyl)-2-[4-(morpholin-4-ylmethyl)benzyl]-4a,5,8,8a-tetrahydro-2H-phthalazin-1-one

(23) cis-4-(3,4-Diethoxyphenyl)-2-[4-(morpholin-4-ylmethyl)benzyl]-4a,5,8,8a-tetrahydro-2H-phthalazin-1-one

(24) cis-4-(3,4-Dimethoxyphenyl)-2-(4-morpholin-4-ylbenzyl)-4a,5,8,8a-tetrahydro-2H-phthalazin-1-one

(25) cis-4-(3-Ethoxy-4-methoxyphenyl)-2-[4-(morpholin-4-ylmethyl)benzyl]-4a,5,8,8a-tetrahydro-2H-phthalazin-1-one

(26) cis-4-(3-Isopropoxy-4-methoxyphenyl)-2-[4-(morpholin-4-ylmethyl)benzyl]-4a,5,8,8a-tetrahydro-2H-phthalazin-1-one

The compound of the invention has asymmetric carbon at the 4a-position and the 8a-position of the phthalazinone skeleton. In case where the configuration is not specifically defined, the invention encompasses geometric isomers (4a,8a-cis isomer, 4a,8a-trans isomer), optical isomers (4aS,8aS isomers, 4aS,8aR isomer, 4aR,8aS isomer, 4aR,8aR isomer), and their mixtures.

The present invention is described below in detail.

"Alkyl" in the present invention includes linear or branched alkyl having 1 to 10 carbon atoms, such as methyl, ethyl, n-propyl, isopropyl, n-butyl, isobutyl, sec-butyl, tert-butyl, n-pentyl, isopentyl, n-hexyl, isohexyl, n-heptyl, isoheptyl, n-octyl, n-nonyl, and n-decyl. In particular, preferred is alkyl having 1 to 6 carbon atoms; and more preferred is alkyl having 1 to 3 carbon atoms.

The alkyl moiety of "alkoxy" includes the above-mentioned alkyl.

"Cycloalkyl" in the present invention includes mono- to tri-cyclic cycloalkyl having 3 to 10 carbon atoms, such as cyclopropyl, cyclobutyl, cyclopentyl, cyclohexyl, cycloheptyl, cyclooctyl, cyclononyl, cyclodecanyl, adamantyl (1-adamantyl, 2-adamantyl, etc.),2-bicyclo[3.1.1.]heptyl, and 2-bicyclo[2.2.1]heptyl. In particular, preferred is monocyclic cycloalkyl having 3 to 8 carbon atoms; and more preferred is monocyclic cycloalkyl having 4 to 6 carbon atoms.

"Phenylene" in the present invention includes, for example, 1,2-phenylene, 1,3-phenylene, and 1,4-phenylene.

"Divalent aromatic heterocyclic group" in the present invention includes one made up of 6 ring-constituting atoms and having 1 or 2 nitrogen atoms as the ring-constituting atoms, for example, the following groups.

[Chemical Formula 6]

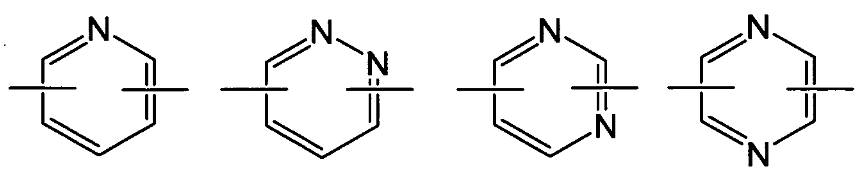

"Alkylene" in the present invention includes linear or branched alkylene having 1 to 6 carbon atoms, for example, the following groups.

[Chemical Formula 7]

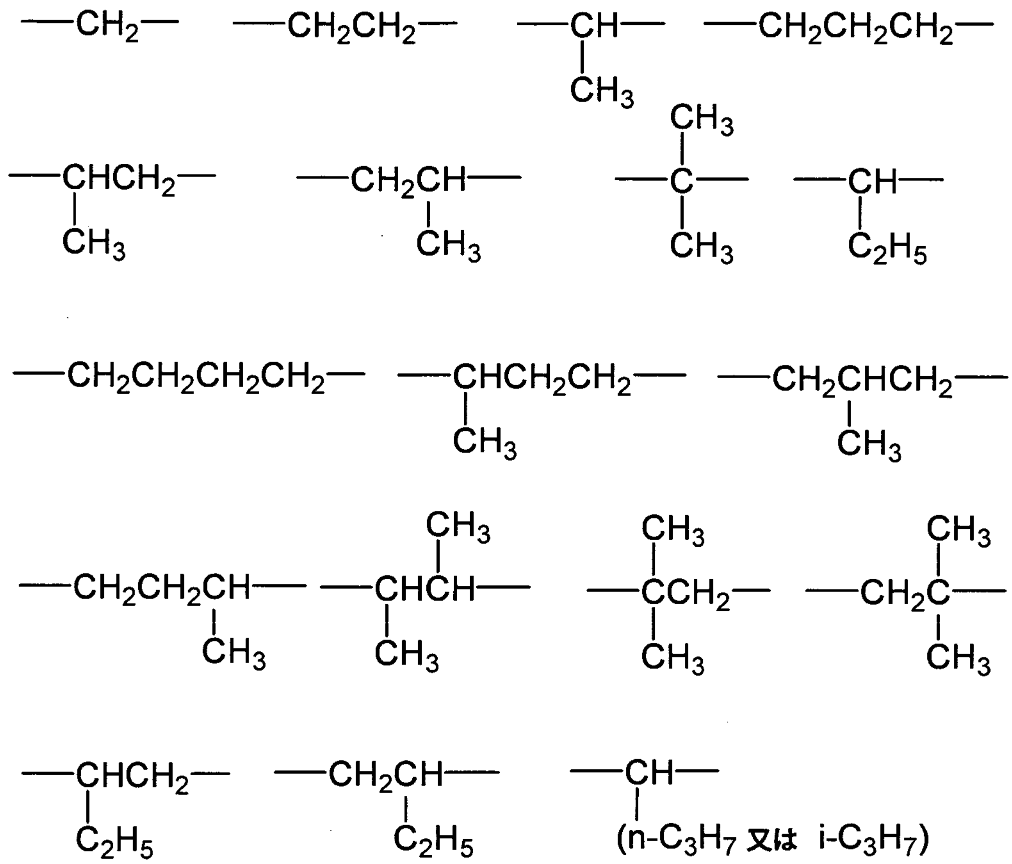

In particular, preferred is alkylene having 1 to 4 carbon atoms; and more preferred is alkylene having 1 to 3 carbon atoms.

"Alkenylene" in the present invention includes linear or branched alkenylene having 2 to 6 carbon atoms, for example, the following groups.

[Chemical Formula 8]

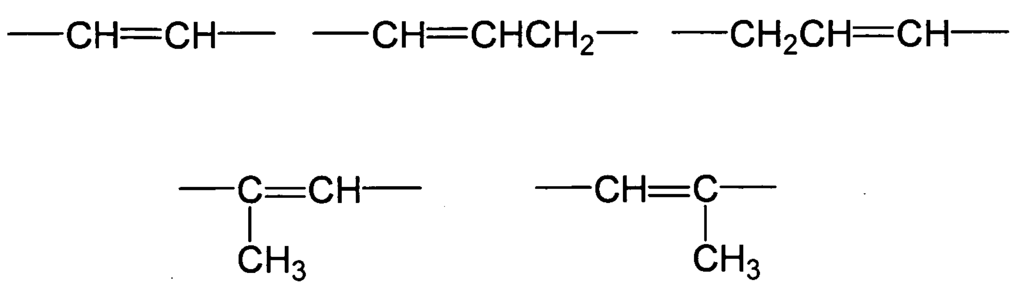

In particular, preferred is alkenylene having 2 to 4 carbon atoms; and more preferred is alkenylene having 2 or 3 carbon atoms.

"Alkynylene" in the present invention includes linear or branched alkynylene having 2 to 6 carbon atoms, for example, the following groups.

[Chemical Formula 9]

$$-C\equiv C- \qquad -C\equiv CCH_2- \qquad -CH_2C\equiv C-$$

In particular, preferred is alkynylene having 2 to 4 carbon atoms; and more preferred is alkynylene having 2 or 3 carbon atoms.

"Saturated or unsaturated cyclic amino group" in the present invention includes mono- to tri-cyclic saturated or unsaturated cyclic amino group which is made up of 5 to 10 ring-constituting atoms and has at least one nitrogen atom, and besides, optionally 1 to 3 nitrogen, oxygen or sulfur atoms as the ring-constituting atoms. The cyclic amino group may be substituted with $R^{31}$ and $R^{32}$, in which the ring-constituting nitrogen atom may form an oxide. Specifically, it includes pyrrolidin-1-yl, piperidin-1-yl, piperazin-1-yl, morpholin-4-yl, 4-oxido-morpholin-4-yl, thiomorpholin-4-yl, imidazol-1-yl, dihydropyridin-1-yl, 1,2,3,6-tetrahydropyridin-1-yl, homopiperazin-1-yl, 2,3-dihydro-1H-indol-1-yl, 1,2,3,4-tetrahydroquinolin-1-yl, 1,2,3,4-tetrahydroisoquinolin-2-yl, and octahydroquinolin-1-yl. The cyclic amino group may be substituted with one or two substituents selected from the group consisting of alkyl, alkoxy, hydroxy, halogen and oxo.

"Halogen" in the present invention includes fluorine, chlorine, bromine, iodine.

[Brief Description of the Drawing]

[Fig. 1]

Fig. 1 shows the effect on dermatitis of a sequential therapy with a test compound and a steroid. The vertical axis indicates the rate of ear swelling (%) ; and the horizontal axis indicates the time (day) . In the drawing, -•- shows the test result of a case where a steroid was applied and then a compound of the invention was applied; -Δ- shows the test result of a case where steroid application was continued; and -□- shows the test result of a case where a steroid was applied and then vaseline was applied.

[Best Mode for Carrying out the Invention]

The compound of the invention can be produced from known compounds or easily synthesizable intermediates, for example, according to the methods mentioned below. In producing the compound of the invention, when the starting material has a substituent that affects the reaction, it is general that the starting material is protected with a suitable protective group according to a known method and then the reaction is performed. The protective group can be removed in an ordinary method after the reaction.

<u>Production Method 1 (method of producing compound [1a] wherein Z is alkylene, alkenylene or alkynylene)</u>

[Chemical Formula 10]

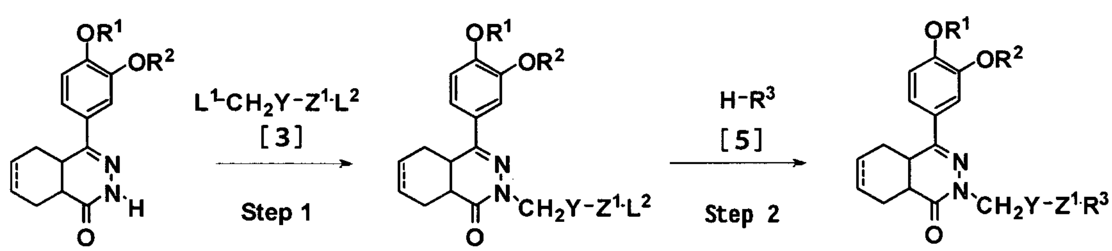


[2] [4] [1 a]

In the above reaction formula, Y, $R^1$, $R^2$ and $R^3$ are the same as the above-mentioned; $Z^1$ represents alkylene, alkenylene or alkynylene; $L^1$ and $L^2$ each represents a leaving group.)

Step 1:

The reaction of a compound [2] and a compound [3] can be attained in an ordinary method (for example, see Patent Reference 1, Non-Patent References 62 and 63). For example, in the presence or absence of a base (e. g. , potassium carbonate, calcium carbonate, sodium carbonate, cesium carbonate, triethylamine, pyridine, 2,6-lutidine, sodium hydride, n-butyllithium, sodium methoxide, and sodium ethoxide), the reaction can be attained at a temperature within the range from -20 to 150˚C. The usable solvent is not specifically limited so far as it has no influence on the reaction, including, for example, ethers such as tetrahydrofuran and diethyl ether; amides such as N,N-dimethylformamide and N,N-dimethylacetamide; nitriles such as acetonitrile and propionitrile; hydrocarbons such as benzene and toluene; halogenohydrocarbons such as chloroform and dichloromethane; alcohols such as methanol, ethanol and isopropanol; and their mixed solvents. The reaction time may vary depending on the type of the starting material and the base and on the reaction temperature, but in general, it may suitably fall within the range from 30 minutes to 48 hours. The amount of the compound [3] and the base to be used may be suitably from 1 to 10 molar times that of the compound [2].
Not specifically limited, the leaving groups $L^1$ and $L^2$ may be, for example, suitably a halide substituent such as chloride, bromide and iodide; a hydroxyl group activated through esterification (e.g., p-toluenesulfonyl group, methanesulfonyl group).

Step 2:

The reaction of the compound [4] obtained in the step 1 and a saturated or unsaturated cyclic amine compound [5] can be attained in an ordinary method. For example, in the presence or absence of a base (e.g., potassium carbonate, calcium carbonate, sodium carbonate, cesium carbonate, triethylamine, pyridine, 2,6-lutidine, sodium hydride, n-butyllithium, sodium methoxide, and sodium ethoxide), the reaction can be attained at a temperature within the range from -20 to 150˚C. The usable solvent is not specifically limited so far as it has no influence on the reaction, including, for example, ethers such as tetrahydrofuran and diethyl ether; amides such as N,N-dimethylformamide and N,N-dimethylacetamide; nitriles such as acetonitrile and propionitrile; hydrocarbons such as benzene and toluene; halogenohydrocarbons such as chloroform and dichloromethane; alcohols such as methanol, ethanol and isopropanol; and their mixed solvents. The reaction time may vary depending on the type of the starting material and the base and on the reaction temperature, but in general, it may suitably fall within the range from 30 minutes to 48 hours. The amount of the compound [5] to be used may be suitably from 1 to 10 molar times that of the compound [4].
The starting compound [2] can be produced, for example, according to the same method as those described in Patent Reference 1 and Non-Patent References 62 to 64.
The starting compound [3] is commercially available, or can be produced, for example, according to the same method as those described in Non-Patent References 65 to 67.

Production Method 2 (method for producing compound [1c] wherein Z is a single bond)

[Chemical Formula 11]

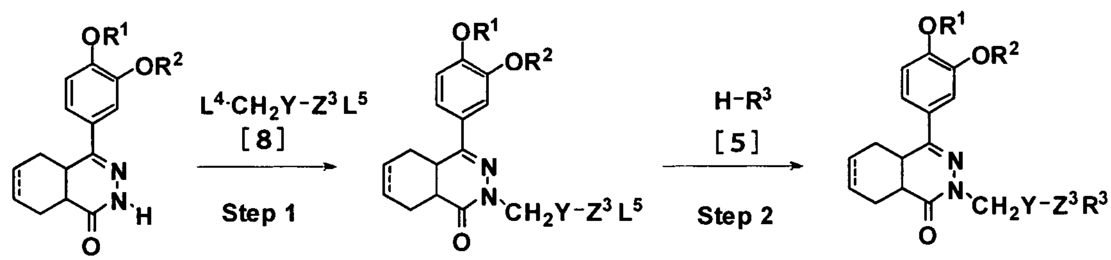


[2] [9] [1 c]

(In the above reaction formula, Y, $R^1$, $R^2$ and $R^3$ are the same as the above-mentioned; $Z^3$ represents a single bond; $L^4$ and $L^5$ each represent a leaving group.)

Step 1:

The reaction of a compound [2] and a compound [8] can be attained in an ordinary method. For example, the product can be produced according to the same method as that of the step 1 of the production method 1.
Not specifically limited, the leaving groups $L^4$ and $L^5$ may be, for example, suitably a halide substituent such as chloride, bromide and iodide; a hydroxyl group activated through esterification (e.g., p-toluenesulfonyl group and methanesulfonyl group).

Step 2:

The reaction of the compound [9] obtained in the step 1 and a saturated or unsaturated cyclic amine compound [5] can be attained in an ordinary method. For example, in the presence or absence of a base (e.g., potassium carbonate, calcium carbonate, sodium carbonate, cesium carbonate, triethylamine, pyridine, 2,6-lutidine, sodium hydride, n-butyllithium, phenyllithium, sodium methoxide, and sodium ethoxide), the reaction can be attained at a temperature within the range from -20 to 200˚C. The product can also be produced according to a method of using a palladium catalyst or a copper catalyst (for example, see Non-Patent References 70 to 72 and 75).
The palladium catalyst includes, for example, tetrakis(triphenylphosphine) palladium, dichlorobistriphenylphosphine palladium, dichlorobis(tri-o-tolylphosphine) palladium and bis(dibenzylideneacetone) palladium; and the copper catalyst includes, for example, copper iodide. The ligand includes tris(2-methylphenyl) phosphine, 1,1'-bis(diphenylphosphino) ferrocene, 2,2'-bis(diphenylphosphino)-1,1'-binaphthyl, N-methylglycine, and L-proline. The usable solvent is not specifically limited so far as it has no influence on the reaction, including, for example, ethers such as tetrahydrofuran and diethyl ether; amides such as N,N-dimethylformamide and N,N-dimethylacetamide; nitriles such as acetonitrile and propionitrile; hydrocarbons such as benzene and toluene; halogenohydrocarbons such as chloroform and dichloromethane; alcohols such as methanol, ethanol, isopropanol and 2-methoxyethanol; dimethyl sulfoxide; and their mixed solvents.
The reaction time may vary depending on the type of the starting material and the base and on the reaction temperature, but in general, it may suitably fall within the range of from 30 minutes to 48 hours. The amount of the compound [5] to be used may be suitabe from 1 to 10 molar times that of the compound [9].
The starting compound [8] is commercially available, or can be produced, for example, according to the same method as those described in Non-Patent Reference 68.
An N-oxide compound of the invention (for example, see Example 21) can be produced by processing the compound obtained in the final step in an ordinary manner. For example, the compound can be oxidized with mCPBA or hydrogen peroxide to produce its N-oxide (for example, see Non-Patent References 87 and 88).
The compound of the invention can be used as a medicament as it is, but it is also possible to use it by converting it into a form of a pharmaceutically acceptable salt by a conventional method. Examples of such a salt may include salts of mineral acids such as hydrochloric acid, hydrobromic acid, sulfuric acid and phosphoric acid and salts of organic acids such as acetic acid, citric acid, tartaric acid, maleic acid, succinic acid, fumaric acid, p-toluenesulfonic acid, benzenesulfonic acid and methanesulfonic acid.
For example, hydrochloride of the compound of the invention can be produced by dissolving the compound of the invention in a solution of hydrogen chloride in alcohol, ethyl acetate or ether.
As shown in the test examples below, the compound of the invention or its pharmaceutically acceptable salt is useful as medicines and is especially effective in inflammatory diseases such as atopic dermatitis.
In a case where the compound of the invention or its pharmaceutically acceptable salt is administered as a medicine, the compound of the invention or its pharmaceutically acceptable salt can be administered to mammals including humans, directly as it is or in the form of a pharmaceutical composition containing it in a pharmaceutically acceptable nontoxic and inert carrier, for example, in a ratio of from 0.0001 to 99.5 %, preferably from 0.001 to 90 %.
One or more auxiliary agent(s) for pharmaceutical formulations such as a solid, semi-solid or liquid diluent, a filler and others may be used. It is desirable that the pharmaceutical composition is administered as a unit dose form. Although there is no particular limitation for the unit dose form of the pharmaceutical composition concerning the present invention, it goes without saying that administration is performed by a dose form which is suitable for the administering method. Topical administration (for example, percutaneous administration and inhalation) is preferred.
The dose of the therapeutical agent for inflammatory diseases including atopic dermatitis is preferably determined in consideration of the characteristic and the degree of the disease, the age, the body weight and the condition of the patient, and the administration method. In general, the dose may be within the range from 0.01 to 1000 mg/human/day, preferably from 0.1 to 500 mg/human/day, in terms of the amount of the effective ingredient of the compound of the invention or its pharmaceutically acceptable salt.
In some cases, the dose less than the above may be sufficient and, in some other cases, the dose more than the above may be necessary. Administration may also be performed by dividing the dose into two to five portions a day.

[Examples]

The present invention will now be described in more detail by way of Reference Examples, Examples, Test Examples and Formulation Examples of the compound of the present invention, to which, however, the present invention is not limited.

Reference Example 1 cis-4-(3,4-Dimethoxyphenyl)-4a,5,8,8a-tetrahydro-2H-phthalazin-1-one

Using cis-2-(3,4-Dimethoxybenzoyl)-1,2,3,6-tetrahydrobenzoic acid and Hydrazine monohydrate, it was produced according to a known method (for example, see Patent Reference 1 or Non-Patent Reference 62).
$^1$H-NMR($CDCl_3$) δ: 2.1-2.3(3H,m), 2.85(1H,t), 3.0(1H,d), 3.4(1H,m), 3.93(3H,s), 3.94(3H,s), 5.76(2H,m), 6.87(1H,d), 7.24(1H,d), 7.47(1H,d), 8.71(1H,s)

Reference Example 2 cis-(+)-4-(3,4-Dimethoxyphenyl)-4a,5,8,8a-tetrahydro-2H-phthalazin-1-one

Using cis-(+)-2-(3,4-Dimethoxybenzoyl)-1,2,3,6-tetrahydrobenzoic acid (see Non-Patent Reference 64) and Hydrazine monohydrate, it was produced according to a known method (for example, see Patent Reference 1 or Non-Patent Reference 64).
Specific rotation $[\alpha]_D^{25}$ = 581.09° (c = 1.0, chloroform)

Reference Example 3 cis-(-)-4-(3,4-Dimethoxyphenyl)-4a,5,8,8a-tetrahydro-2H-phthalazin-1-one

Using cis-2-(-)-(3,4-Dimethoxybenzoyl)-1,2,3,6-tetrahydrobenzoic acid (see Non-Patent Reference 64) and Hydrazine monohydrate, it was produced according to a known method (for example, see Patent Reference 1 or Non-Patent Reference 64).
Specific rotation $[\alpha]_D^{25}$ = -665.79° (c = 1.0, chloroform)

Reference Example 4 cis-4-(3,4-Dimethoxyphenyl)-4a,5,6,7,8,8a-hexahydro-2H-phthalazin-1-one

Using cis-[2-(3,4-Dimethoxybenzoyl)] cyclohexanecarboxylic acid and Hydrazine monohydrate, it was produced according to a known method (for example, see Patent Reference 1 or Non-Patent Reference 62).
$^1$H-NMR($CDCl_3$) δ: 1.3-1.9(7H,m), 2.55(1H,m), 2.76(1H,bs), 3.13(1H,m), 3.92(3H,s), 3.94(3H,s), 6.86(1H,d), 7.20(1H, dd), 7.45(1H,d), 8.5(1H,s)

Reference Example 5 cis-4-(3-Cyclopentyloxy-4-methoxyphenyl)-4a,5,8,8a-tetrahydro-2H-phthalazin-1-one

Using cis-2-(3-Cyclopentyloxy-4-methoxybenzoyl)-1,2,3,6-tetrahydrobenzoic acid and Hydrazine monohydrate, it was produced according to a known method (for example, see Patent Reference 1 or Non-Patent Reference 63).
Melting point: 153-155°C(melt)

Reference Example 6 trans-4-(3,4-Dimethoxyphenyl)-4a,5,6,7,8,8a-hexahydro-2H-phthalazin-1-one

Using trans-[2-(3,4-Dimethoxybenzoyl)] cyclohexanecarboxylic acid and Hydrazine monohydrate, it was produced according to a known method (for example, see Patent Reference 1 or Non-Patent Reference 62).
$^1$H-NMR($CDCl_3$) δ: 1.0-1.5(4H,m), 1.8-1.95(2H,m), 2.1-2.25(2H,m), 2.4-2.7(2H,m), 3.91(6H,s), 6.88(3H,s), 8.56(1H,s)

Reference Example 7 cis-4-(3,4-Diethoxyphenyl)-4a,5,8,8a-tetrahydro-2H-phthalazin-1-one

Using cis-3-[6-(3,4-Diethoxybenzoyl)] cyclohexenylcarboxylic and Hydrazine monohydrate, it was produced according to a known method (for example, see Patent Reference 1 or Non-Patent Reference 63).
$^1$H-NMR($CDCl_3$) δ: 1.45(6H,t), 2.1-2.6(3H,m), 2.84(1H,t), 2.98(1H,bd), 3.32-3.41(1H,m), 4.13(4H,dd), 5.74(2H,m), 6.86 (1H,d), 7.19(1H,dd), 7.44(1H,d), 8.84(1H,s)

Example 1 cis-4-(3,4-Dimethoxyphenyl)-2-[4-(morpholin-4-ylmethyl)benzyl]-4a,5,8,8a-tetrahydro-2H-phthalazin-1-one

Step 1 cis-2-(4-Bromomethylbenzyl)-4-(3,4-dimethoxyphenyl) -4a,5,8,8a-tetrahydro-2H-phthalazin-1-one

After 6.33 g of cis-4-(3,4-Dimethoxyphenyl)-4a,5,8,8a-tetrahydro-2H-phthalazin-1-one obtained in Reference Example 1 was dissolved in 63 mL of N,N-Dimethylformamide, 1.06 g of Sodium hydride was added thereto under cooling with ice water. Stirred at room temperature for 45 minutes, the suspension was added dropwise to an N,N-Dimethylformamide (63 mL) solution of 29.2 g of 1,4-bis-Bromomethylbenzene, taking 15 minutes, and then stirred at room temperature for 1.5 hours. This was poured into 1.5 L of ice water and was subjected to extraction procedure with Ethyl acetate three times. The organic layers were combined, washed with water and saturated saline, dried with anhydrous Magnesium sulfate, and the solvent was evaporated in vacuo. The crude product was purified through silica gel column chromatography to obtain 5.81 g of the objective compound as a colorless amorphous substance.
$^1$H-NMR($CDCl_3$) δ: 1.95-2.30(3H,m), 2.82(1H,t), 3.0(1H,d), 3.34(1H,ddd), 3.92(6H,s), 4.46(2H,s), 5.03(2H,dd), 5.73(2H,m), 6.85(1H,d), 7.0-7.5(6H,m)

Step 2 cis-4-(3,4-Dimethoxyphenyl)-2-[4-(morpholin-4-ylmethyl)benzyl]-4a,5,8,8a-tetrahydro-2H-phthalazin-1-one

After 5.72 g of cis-2-(4-Bromomethylbenzyl)-4-(3,4-dimethoxyphenyl)-4a,5,8,8a-tetrahydro-2H-phthalazin-1 -one obtained in the step 1 and 2.52 g of Potassium carbonate were added to anhydrous Tetrahydrofuran (122 mL), 1.84 mL of Morpholine was added thereto. Stirred at room temperature for 21 hours, the insoluble matter was filtered through filter paper and the solvent was evaporated in vacuo. The residue was purified through silica gel column chromatography to obtain 5.51 g of the objective compound as a white amorphous substance.
$^1$H-NMR($CDCl_3$) δ: 1.95-2.3(3H,m), 2.42(4H,t), 2.82(1H,t), 3.04(1H,d), 3.35(1H,ddd), 3.46(2H,s), 3.69(4H,t), 3.92(3H,s), 3.93(3H,s), 5.04(2H,dd), 5.72(2H,m), 6.85(1H,d), 7.2-7.37(5H,m), 7.45(1H,d)

| Elemental Analysis value (as $C_{28}H_{33}N_3O_4 \cdot 0.2H_2O$) | | | |
|---|---|---|---|
| Calculated (%) : | C: 70.18 | H: 7.03 | N: 8.77 |
| Found (%) : | C: 70.12 | H: 7.10 | N: 8.75 |

Example 2-1 cis-(+)-4-(3,4-Dimethoxyphenyl)-2-[4-(morpholin-4-ylmethyl)benzyl]-4a,5,8,8a-tetrahydro-2H-phthalazin-1-one

Step 1 cis-(+)-2-(4-Bromomethylbenzyl)-4-(3,4-dimethoxyphenyl)-4a,5,8,8a-tetrahydro-2H-phthalazin-1-one

The objective compound was obtained according to the method of the step 1 in Example 1 but using cis-(+)-4-(3,4-Dimethoxyphenyl)-4a,5,8,8a-tetrahydro-2H-ph thalazin-1-one (Reference Example 2) in place of cis-4-(3,4-Dimethoxyphenyl)-4a,5,8,8a-tetrahydro-2H-phthal azin-1-one.
$^1$H-NMR($CDCl_3$) δ: 1.95-2.30(3H,m), 2.82(1H,t), 3.0(1H,d), 3.34(1H,ddd), 3.92(6H,s), 4.46(2H,s), 5.03(2H,dd), 5.73(2H,m), 6.85(1H,d), 7.0-7.5(6H,m)
Specific rotation $[\alpha]_D^{25}$ = 385.79°(c = 1.0, chloroform)

Step 2 cis-(+)-4-(3,4-Dimethoxyphenyl)-2-[4-(morpholin-4-ylmethyl)benzyl]-4a,5,8,8a-tetrahydro-2H-phthalazin-1-one

The objective compound was obtained according to the method of the step 2 in Example 1 but using cis-(+)-2-(4-Bromomethylbenzyl)-4-(3,4-dimethoxyphenyl)-4a,5,8,8a-tetrahydro-2H-phthalazin-1-one obtained in the step 1 in place of cis-2-(4-Bromomethylbenzyl)-4-(3,4-dimethoxyphenyl)-4a,5,8,8a-tetrahydro-2H-phthalazin-1 -one.
$^1$H-NMR($CDCl_3$) δ: 1.95-2.3(3H,m), 2.42(4H,t), 2.82(1H,t), 3.04(1H,d), 3.35(1H,ddd), 3.46(2H,s), 3.69(4H,t), 3.92(3H,s), 3.93(3H,s), 5.04(2H,dd), 5.72(2H,m), 6.85(1H,d), 7.2-7.37(5H,m), 7.45(1H,d)
Specific rotation $[\alpha]_D^{25}$ = 397.59°(c = 1.0, chloroform)

Example 2-2 cis-(+)-4-(3,4-Dimethoxyphenyl)-2-[4-(morpholin-4-ylmethyl)benzyl]-4a,5,8,8a-tetrahydro-2H-phthalazin-1-one monomaleate

After 49 mg of Maleic acid was added to a 2-Propyl alcohol (2 mL) solution of 200 mg of cis-(+)-4-(3,4-Dimethoxyphenyl)-2-[4-(morpholin-4-ylmethyl)benzyl]-4a,5,8,8a-tetrahydro-2H-phthalazin-1-one obtained in the step 2 in Example 2-1 with stirring under heat, and a small amount of ethanol was added thereto. Stirred at room temperature, this

was evaporated in vacuo to obtain 103 mg of the objective compound as a white powder.

| Elemental Analysis value (as $C_{32}H_{37}N_3O_8$) | | | |
|---|---|---|---|
| Calculated (%): | C: 64.96 | H: 6.30 | N: 7.10 |
| Found (%) : | C: 64.66 | H: 6.19 | N: 7.04 |

Example 3 cis-(-)-4-(3,4-Dimethoxyphenyl)-2-[4-(morpholin-4-ylmethyl)benzyl]-4a,5,8,8a-tetrahydro-2H-phthalazin-1-one

The objective compound was obtained according to the method of Example 1 but using cis-(-)-4-(3,4-Dimethoxyphenyl)-4a,5,8,8a-tetrahydro-2H-phthalazin-1-one (Reference Example 3) in place of cis-4-(3,4-Dimethoxyphenyl)-4a,5,8,8a-tetrahydro-2H-phthalazin-1-one. $^1$H-NMR($CDCl_3$) δ: 1.95-2.3(3H,m), 2.42(4H,t), 2.82(1H,t), 3.04(1H,d), 3.35 (1H,ddd), 3.46(2H,s), 3.69(4H,t), 3.92(3H,s), 3.93(3H,s), 5.04(2H,dd), 5.72(2H,m), 6.85(1H,d), 7.2-7.37(5H,m), 7.45 (1H,d)
Specific rotation $[\alpha]_D^{25}$ = -419.19°(c = 1.0, chloroform)

Example 4 cis-4-(3,4-Dimethoxyphenyl)-2-[4-(piperidin-1-ylmethyl)benzyl]-4a,5,8,8a-tetrahydro-2H-phthalazin-1-one

The objective compound was obtained as a white amorphous substance according to the method of the step 2 in Example 1 but using Piperidine in place of Morpholine.
$^1$H-NMR ($CDCl_3$) δ: 1.4-1.62(6H,m), 1.9-2.37(8H,m), 2.82(1H,t), 3.03(1H,bd), 3.33(1H,ddd), 3.45(2H,s), 3.91(3H,s), 3.92(3H,s), 5.02(2H,dd), 5.70(2H,m), 6.85(1H,d), 7.20-7.36(5H,m), 7.45(1H,d)

Example 5 cis-4-(3,4-Dimethoxyphenyl)-2-[4-(1,4-dioxa-8-azaspiro[4,5]dec-8-ylmethyl)benzyl]-4a,5,8,8a-tetrahydro-2 H-phthalazin-1-one

The objective compound was obtained as a white amorphous substance according to the method of the step 2 in Example 1 but using 1,4-Dioxa-8-azaspiro[4,5]decane in place of Morpholine.
$^1$H-NMR($CDCl_3$) δ: 1.6-1.8(4H,m), 1.9-2.3(3H,m), 2.50(4H,t), 2.82(1H,t), 3.02(1H,bd), 3.36(1H,ddd), 3.48(2H,s), 3.92 (10H,m), 5.02(2H,dd), 5.72(2H,m), 6.85(1H,d), 7.2-7.36(5H,m), 7.45(1H,d)

| Elemental Analysis value (as $C_{31}H_{37}N_3O_5 \cdot 0.5H_2O$) | | | |
|---|---|---|---|
| Calculated (%): | C: 68.87 | H: 7.08 | N: 7.77 |
| Found (%) : | C: 68.72 | H: 6.80 | N: 7.73 |

Example 6 cis-4-(3,4-Dimethoxyphenyl)-2-[4-(4-oxopiperidin-1-ylmethyl)benzyl]-4a,5,8,8a-tetrahydro-2H-phthalazin-1-one

The objective compound was obtained as a white amorphous substance according to the method of the step 2 in Example 1 but using 4-Piperidone hydrochloride monohydrate in place of Morpholine.
$^1$H-NMR ($CDCl_3$) δ: 1.9-2.3(3H,m), 2.47(4H,t), 2.72 (4H, t), 2.83(1H,t), 3.02(1H,d), 3.34(1H,ddd), 3.58(2H,s), 3.92(3H, s), 3.93(3H,s), 5.05(2H,dd), 5.72(2H,m), 6.86(1H,d), 7.22-7.39(5H,m), 7.46(1H,d)

Example 7 cis-4-(3,4-Dimethoxyphenyl)-2-[4-(trans-2,6-dimethylmorpholin-4-ylmethyl)benzyl]-4a,5,8,8a-tetrahydro-2H-phthalazin-1-one

The objective compound was obtained as a white amorphous substance according to the method of the step 2 in Example 1 but using 2,6-Dimethylmorpholine in place of Morpholine.
$^1$H-NMR($CDCl_3$) δ: 1.2(6H,d), 1.9-2.3(4H,m), 2.43(2H,dd), 2.82(1H,t), 3.02(1H,d), 3.27-3.40(3H,m), 3.92(3H,s), 3.93 (3H,s), 4.0(2H,m), 5.07(2H,dd), 5.72(2H,m), 6.86(1H,d), 7.21-7.36(5H,m), 7.46(1H,d)

Example 8 cis-4-(3,4-Dimethoxyphenyl)-2-[4-(cis-2,6-dimethylmorpholin-4-ylmethyl)benzyl]-4a,5,8,8a-tetrahydro-2H-phthalazin-1-one

The objective compound was obtained as a white amorphous substance according to the method of Example 7.
$^1$H-NMR($CDCl_3$) δ: 1.11(6H,d), 1.73(2H,t), 1.9-2.3(3H,m), 2.68(2H,d), 2.85(1H,t), 3.03(1H,d), 3.37(1H,m), 3.45(2H,s),

3. 66 (2H,m), 3.92(3H,s), 3.93(3H,s), 5.05(2H,dd), 5.72 (2H, m), 6.86(1H,d), 7.21-7.37(5H,m), 7.45(1H,d)

Example 9 cis-4-(3,4-Dimethoxyphenyl)-2-[4-(imidazol-1-ylmethyl)benzyl]-4a,5,8,8a-tetrahydro-2H-phthalazin-1-one

30 mg of Sodium hydride was added to an N,N-Dimethylformamide (1 mL) solution of Imidazole under cooling with ice water and the solution was stirred for 10 minutes. Next, an N,N-Dimethylformamide (2 mL) solution of 230 mg of cis-2-(4-Bromomethylbenzyl)-4-(3,4-dimethoxyphenyl)-4a,5,8,8a-tetrahydro-2H-phthalazin-1-one obtained in the step 1 in Example 1 was added thereto dropwise and stirred for 10 minutes. Water was added to the reaction liquid, and the reaction liquid was subjected to extraction procedure with Ethyl acetate. The organic layers were combined, washed with water and saturated saline and dried with Magnesium sulfate, and the solvent was evaporated in vacuo. The crude product was purified through silica gel column chromatography to obtain 110 mg of the objective compound as a white amorphous substance.
$^1$H-NMR($CDCl_3$) δ: 1.8-2.3(3H,m), 2.81(1H,t), 3.02(1H,d), 3.35(1H,ddd), 3.92(6H,s), 5.00(2H,dd), 5.08(2H,s), 5.72(2H, m), 6.86(1H,d), 6.88(1H,s), 7.07-7.11(3H,m), 7.23(1H,dd), 7.36-7.44(3H,m), 7.52(1H,s)

Example 10 cis-4-(3,4-Dimethoxyphenyl)-2-[3-(morpholin-4-ylmethyl)benzyl]-4a,5,8,8a-tetrahydro-2H-phthalazin-1-one

Step 1 cis-2-(3-Bromomethylbenzyl)-4-(3,4-dimethoxyphenyl)-4a,5,8,8a-tetrahydro-2H-phthalazin-1-one

The objective compound was obtained according to the method of the step 1 in Example 1 but using 1,3-Bis-bromomethylbenzene in place of 1,4-Bis-bromomethylbenzene.
$^1$H-NMR($CDCl_3$) δ: 1.9-2.30(3H,m), 2.83(1H,t), 3.04(1H,d), 3.34(1H,ddd), 3.92(3H,s), 3.93(3H,s), 4.47(2H,s), 5.03(2H, dd), 5.73(2H,m), 6.86(1H,d), 7.2-7.44(6H,m)

Step 2 cis-4-(3,4-Dimethoxyphenyl)-2-[3-(morpholin-4-ylmethyl)benzyl]-4a,5,8,8a-tetrahydro-2H-phthalazin-1-one

The objective compound was obtained as a white amorphous substance according to the method of the step 2 in Example 1 but using cis-2-(3-Bromomethylbenzyl)-4-(3,4-dimethoxyphenyl)-4a,5,8,8a-tetrahydro-2H-phthalazin-1-one obtained in the step 1 in place of cis-2-(4-Bromomethylbenzyl)-4-(3,4-dimethoxyphenyl)-4a,5,8,8a-tetrahydro-2H-phthalazin-1-one.
$^1$H-NMR($CDCl_3$) δ: 1.80-2.3(3H,m), 2.39(4H,t), 2.83(1H,t), 3.04(1H,d), 3.37(1H,ddd), 3.46(2H,s), 3.64(4H,t), 3.92(6H, s), 5.03(2H,dd), 5.72(2H,m), 6.85(1H,d), 7.21-7.44(5H,m), 7.44(1H,d)

Example 11 cis-4-(3,4-Dimethoxyphenyl)-2-[2-(morpholin-4-ylmethyl)benzyl]-4a,5,8,8a-tetrahydro-2H-phthalazin-1-one

Step 1 cis-2-(2-Bromomethylbenzyl)-4-(3,4-dimethoxyphenyl)-4a,5,8,8a-tetrahydro-2H-phthalazin-1-one

The objective compound was obtained as a white amorphous substance according to the method of the step 1 in Example 1 but using 1,2-Bis-bromomethylbenzene in place of 1,4-Bis-bromomethylbenzene.
$^1$H-NMR($CDCl_3$) δ: 1.8-2.30(3H,m), 2.84(1H,t), 3.05(1H,d), 3.34(1H,ddd), 3.92(3H,s), 3.93(3H,s), 4.85(2H,dd), 5.2(2H, dd), 5.73(2H,m), 6.86(1H,d), 7.2-7.5(6H,m)

Step 2 cis-4-(3,4-Dimethoxyphenyl)-2-[2-(morpholin-4-ylmethyl)benzyl]-4a,5,8,8a-tetrahydro-2H-phthalazin-1-one

The objective compound was obtained as a white amorphous substance according to the method of the step 2 in Example 1 but using cis-2-(2-Bromomethylbenzyl)-4-(3,4-dimethoxyphenyl)-4a,5,8,8a-tetrahydro-2H-phthalazin-1-one obtained in the step 1 in place of cis-2-(4-Bromomethylbenzyl)-4-(3,4-dimethoxyphenyl)-4a,5,8,8a-tetra hydro-2H-phthalazin-1-one.
$^1$H-NMR($CDCl_3$) δ: 1.90-2.3(3H,m), 2.46(4H,t), 2.82(1H,t), 3.03(1H,d), 3.35(1H,ddd), 3.65-3.7(6H,m), 3.90(3H,s), 3.92 (3H,s), 5.25(2H,dd), 5.72(2H,m), 6.85(1H,d), 7.2-7.27(4H,m), 7.38-7.41(2H,m)

Example 12 cis-4-(3,4-Dimethoxyphenyl)-2-[6-(morpholin-4-ylmethyl)pyridin-2-ylmethyl]-4a,5,8,8a-tetrahydro-2H-phthalazin-1-one

Step 1 cis-2-(6-Bromomethylpyridin-2-ylmethyl)-4-(3,4-dimethoxyphenyl)-4a,5,8,8a-tetrahydro-2H-phthalazin-1-one

The objective compound was obtained according to the method of the step 1 in Example 1 but using 2,6-Bis-bromomethylpyridine (for example, see Non-Patent Reference 73) in place of 1,4-Bis-bromomethylbenzene.
$^1$H-NMR($CDCl_3$) δ: 2.15-2.4(2H,m) , 2.8-3.1(3H,m), 3.4(1H,m), 3.89(3H,s), 3.91(3H,s), 4.51(2H,s), 5.22(2H,dd), 5.75 (2H,m), 6.88(1H,d), 7.12(1H,d), 7.2-7.3(2H,m), 7.43(1H,dd), 7.63(1H,t)

Step 2 cis-4-(3,4-Dimethoxyphenyl)-2-[6-(morpholin-4-ylmethyl)pyridin-2-ylmethyl]-4a,5,8,8a-tetrahydro-2H-phtha lazin-1-one

The objective compound was obtained as a white amorphous substance according to the method of the step 2 in Example 1 but using cis-2-(6-Bromomethylpyridin-2-ylmethyl)-4-(3,4-dimethoxyphenyl)-4a,5,8,8a-tetrahydro-2H-phthalazin-1-one obtained in the step 1 in place of cis-2-(4-bromomethylbenzyl)-4-(3,4-dimethoxyphenyl)-4a,5,8,8a-tetrahydro-2H-phthalazin-1-one.
$^1$H-NMR($CDCl_3$) δ: 2.1-2.4(3H,m),2.49(4H,t), 2.92(1H,t), 3.05(1H,d), 3.40(1H,ddd), 3.62(2H,s), 3.68(4H,t), 3.88(3H,s), 3.91(3H, s), 5.20(2H,dd), 5.76(2H,m), 6.85(1H,d), 7.07(1H,d), 7.2-7.31(2H,m), 7.40(1H,d), 7.59(1H,t)

| Elemental Analysis value (as $C_{27}H_{32}N_4O_4 \cdot H_2O$) | | | |
|---|---|---|---|
| Calculated (%): | C: 65.57 | H: 6.93 | N: 11.33 |
| Found (%) : | C: 65.65 | H: 6.56 | N: 11.30 |

Example 13 cis-4-(3,4-Dimethoxyphenyl)-2-[4-(morpholin-4-ylmethyl)benzyl]-4a,5,6,7,8,8a-hexahydro-2H-phthalazin-1-one

Step 1 cis-2-(4-Bromomethylbenzyl)-4-(3,4-dimethoxyphenyl)-4a,5,6,7,8,8a-hexahydro-2H-phthalazin-1-one

The objective compound was obtained as a colorless oil according to the method of the step 1 in Example 1 but using cis-4-(3,4-Dimethoxyphenyl)-4a,5,6,7,8,8a-hexahydro-2H-pht halazin-1-one obtained in Reference Example 4 in place of cis-4-(3,4-Dimethoxyphenyl)-4a,5,8,8a-tetrahydro-2H-phthalazin-1-one.
$^1$H-NMR($CDCl_3$) δ: 1.2-1.9(7H,m), 2.57(1H,d), 2.73(1H,s), 3.09(1H,m), 3.91(6H,s), 4.47(2H,s), 5.03(2H,dd), 6.85(1H, d), 7.17(6H,m)

Step 2 cis-4-(3,4-Dimethoxyphenyl)-2-(4-morpholin-4-ylmethylbenzyl)-4a,5,6,7,8,8a-hexahydro-2H-phthalazin-1-one

The objective compound was obtained as a white amorphous substance according to the method of the step 2 in Example 1 but using cis-2-(4-Bromomethylbenzyl)-4-(3,4-dimethoxyphenyl)-4a,5,6,7,8,8a-hexahydro-2H-phthalazin-1-one obtained in the step 1 in place of cis-2-(4-Bromomethylbenzyl)-4-(3,4-dimethoxyphenyl)-4a,5,8,8a-tetra hydro-2H-phthalazin-1-one.
$^1$H-NMR($CDCl_3$) δ: 1.2-1.9(7H,m), 2.42(4H,t), 2.55(1H,m), 2.73(1H,m), 3.08(1H,m), 3.46(2H,s), 3.69(4H,t), 3.91(6H,s), 5.03(2H,dd), 6.85(1H,d), 7.2-7.45(6H,m)

| Elemental Analysis value (as $C_{28}H_{35}N_3O_4 \cdot 0.2H_2O$) | | | |
|---|---|---|---|
| Calculated (%): | C:69.89 | H:7.41 | N:8.73 |
| Found (%) : | C:69.75 | H:7.20 | N:8.57 |

Example 14 cis-4-(3-Cyclopentyloxy-4-methoxyphenyl)-2-[4-(morpholin-4-ylmethyl)benzyl]-4a,5,8,8a-tetrahydro-2H-phthalazin-1-one

Step 1 cis-2-(4-Bromomethylbenzyl)-4-(3-cyclopentyloxy-4-methoxyphenyl)-4a,5,8,8a-tetrahydro-2H-phthalazin-1-one

The objective compound was obtained as a white amorphous substance according to the method of the step 1 in Example 1 but using cis-4-(3-Cyclopentyloxy-4-methoxyphenyl)-4a,5,8,8a-tetrahydro-2H-phthalazin-1-one obtained in Reference Example 5 in place of cis-4-(3,4-Dimethoxyphenyl)-4a,5,8,8a-tetrahydro-2H-phthalazin-1-one.

$^1$H-NMR($CDCl_3$) δ: 1.55-2.3(11H,m), 2.82(1H,t), 3.1(1H,d), 3.35(1H,ddd), 3.88(3H,s), 4.47(2H,s), 4.75-4.9(1H,m), 5.0 (2H,dd), 5.75(2H,m), 6.84(1H,d), 7.18-7.43(6H,m)

Step 2 cis-4-(3-Cyclopentyloxy-4-methoxyphenyl)-2-[4-(morpholin-4-ylmethyl)benzyl]-4a,5,8,8a-tetrahydro-2H-phthalazin-1-one

The objective compound was obtained as a white amorphous substance according to the method of the step 2 in Example 1 but using cis-2-(4-Bromomethylbenzyl)-4-(3-cyclopentyloxy-4-methoxyphenyl)-4a,5,8,8a-tetrahydro-2H-phthalazin-1-one obtained in the step 1 in place of cis-2-(4-Bromomethylbenzyl)-4-(3,4-dimethoxyphenyl)-4a,5,8,8a-tetrahydro-2H-phthalazin-1-one.
$^1$H-NMR($CDCl_3$) δ: 1.6-2.3(11H,m), 2.42(4H,t), 2.8(1H,t), 3.03(1H,d), 3.30(1H,ddd), 3.45(2H,s), 3.69(4H,t), 3.87(3H,s), 4.80(1H,m), 5.0(2H,dd), 5.72(2H,m), 6.83(1H,d), 7.19-7.45(6H,m)

| Elemental Analysis value (as $C_{32}H_{39}N_3O_4$) | | | |
|---|---|---|---|
| Calculated (%): | C: 72.56 | H: 7.42 | N: 7.93 |
| Found (%) : | C: 72.31 | H: 7.16 | N: 7.96 |

Example 15 cis-(+)-4-(3,4-Dimethoxyphenyl)-2-[4-(2-oxo-piperidin-1-ylmethyl)benzyl]-4a,5,8,8a-tetrahydro-2H-phthalazin-1-one

16 mg of Sodium hydride was added to an N,N-Dimethylformamide (2 mL) solution of 36 mg of 2-Piperidone under cooling with ice water and the solution was stirred for 15 minutes at room temperature. Next, an N,N-Dimethylformamide (4 mL) solution of 154 mg of cis-(+)-2-(4-Bromomethylbenzyl)-4-(3,4-dimethoxyphenyl)-4a,5,8,8a-tetrahydro-2H-phthalazin -1-one obtained in the step 1 in Example 2-1 was added thereto deopwise under cooling with ice water and stirred for 1 hour. This was returned to room temperature and then stirred for 2 hours. A few drops of 1 N Hydrochloric acid were added to the reaction liquid, and the reaction liquid was diluted with Ethyl acetate. The organic layer was washed with water and saturated saline and dried with anhydrous Magnesium sulfate, and the solvent was evaporated in vacuo. The crude product was purified through silica gel column chromatography to obtain 55 mg of the objective compound as a white amorphous substance.
$^1$H-NMR($CDCl_3$) δ: 1.7-1.9(4H,m), 1.9-2.3(3H,m), 2.45(2H,t), 2.82(1H,t), 3.02(1H,bd), 3.18(2H,t), 3.34(1H,ddd), 3.92 (3H,s), 3.93(3H,s), 4.56(2H,s), 5.02(2H,dd), 5.72(2H,m), 6.86(1H,d),7.16-7.46(6H,m)
Specific rotation $[\alpha]_D^{25}$ = 503.40˚(c = 1.0, chloroform)

Example 16 cis-(+)-4-(3,4-Dimethoxyphenyl)-2-[4-(3-oxopiperazin-1-ylmethyl)benzyl]-4a,5,8,8a-tetrahydro-2H-phthalazin-1-one

After 152 mg of cis-(+)-2-(4-Bromomethylbenzyl)-4-(3,4-dimethoxyphenyl)-4a,5,8,8a-tetrahydro-2H-phthalazin-1 -one obtained in the step 1 in Example 2-1 was dissolved in 3 mL of Tetrahydrofuran, 162 mg of 2-Piperazinone and 224 mg of Potassium carbonate were added thereto and then the reaction liquid was stirred at room temperature for 6 hours. Water was added to the reaction liquid, and the reaction liquid was subjected to extraction procedure with ethyl acetate. The organic layer was washed with water and saturated saline and dried with anhydrous Magnesium sulfate, and the solvent was evaporated in vacuo. The crude product was purified through silica gel column chromatography to obtain 157 mg of the objective compound as a white amorphous substance.
$^1$H-NMR($CDCl_3$) δ: 1.9-2.3(3H,m), 2.62(2H,t), 2.82(1H,t), 3.02(1H,bd), 3.14(2H,s), 3.3-3.5(3H,m), 3.54(2H,s), 3.92 (3H, s), 3.94 (3H, s), 5.02 (2H,dd), 5.73(2H,m), 5.94(1H,bs), 6.86(1H,s), 7.2-7.3(3H,m), 7.3-7.4(2H,m), 7.46(1H,d)
Specific rotation $[\alpha]_D^{25}$ = 432.49 (c = 1.0, chloroform)

Example 17 cis-(+)-4-(3,4-Dimethoxyphenyl)-2-[4-(1,1-dioxidothiomorpholin-4-ylmethyl)benzyl]-4a,5,8,8a-tetrahydro-2H-phthalazin-1-one

After 158 mg of cis-(+)-2-(4-Bromomethylbenzyl)-4-(3,4-dimethoxyphenyl)-4a,5,8,8a-tetrahydro-2H-phthalazin-1 -one obtained in the step 1 in Example 2-1 was dissolved in 20 mL of N,N-Dimethylformamide, 1.10 g of 1,1,-Dioxidothiomorpholine hydrochloride and 1.77 g of Potassium carbonate were added thereto and then the reaction liquid was stirred at room temperature for 9 hours. Water was added to the reaction liquid, and the reaction liquid was subjected to extraction procedure with ethyl acetate. The organic layer was washed with water and saturated saline and dried with anhydrous Magnesium sulfate, and the solvent was evaporated in vacuo. The crude product was purified through silica gel column chromatography to obtain 827 mg of the objective compound as a white amorphous substance.

$^1H$-NMR($CDCl_3$) δ: 1.9-2.3(3H,m), 2.83(1H,t), 2.9-3.1(9H,m), 3.35(1H,ddd), 3.61(2H,s), 3.92(3H,s), 3.93(3H,s), 5.02 (2H,dd), 5.73(2H,m), 6.86(1H,d), 7.2-7.3(3H,m), 7.36(2H,d), 7.46(1H,d)
Specific rotation $[\alpha]_D^{25}$ = 418.59° (c = 1.0, chloroform)

Example 18 cis-(+)-4-(3,4-Dimethoxyphenyl)-2-[4-(4-methyl-3-oxopiperazin-1-ylmethyl)benzyl]-4a,5,8,8a-tetrahydro-2H- phthalazin-1-one

After 98 mg of cis-(+)-4-(3,4-Dimethoxyphenyl)-2-[4-(3-oxopiperazin-1-ylmethyl)benzyl]-4a,5,8,8a-tetrahydro -2H-phthalazin-1-one obtained in Example 16 was dissolved in 2 mL of N, N-Dimethylformamide, 10 mg of Sodium hydride was added thereto under cooling with ice water and the reaction liquid was stirred at room temperature for 30 minutes. Next, 14 μL of methyl iodide was added dropwise thereto under cooling with ice water and stirred for 1 hour. A small amount of 1 N Hydrochloric acid was added to the reaction liquid, and the reaction liquid was diluted with Ethyl acetate. The organic layer was washed with water and saturated saline and dried with anhydrous Magnesium sulfate, and the solvent was evaporated in vacuo. The crude product was purified through silica gel column chromatography to obtain 26 mg of the objective compound as a white amorphous substance.
$^1H$-NMR($CDCl_3$) δ: 1.9-2.3(3H,m), 2.64(2H,t), 2.82(1H,t), 2.93(3H,s), 3.02(1H,bd), 3.13(2H,s), 3.2-3.4(3H,m), 3.51(2H, s), 3.92(3H,s), 3.93(3H,s), 5.02(2H,dd), 5.73(2H,m), 6.86(1H,d), 7.2-7.3(3H,m), 7.35(2H,d), 7.46(1H,d)
Specific rotation $[\alpha]_D^{25}$ = 443.19° (c = 1.0, chloroform)

Example 19 cis-(+)-4-(3,4-Dimethoxyphenyl)-2-[4-(2,6-dioxo-piperidin-1-ylmethyl)benzyl]-4a,5,8,8a-tetrahydro-2H-phthalazin-1-one

The objective compound was obtained as a white amorphous substance according to the method of Example 15 but using Glutarimide in place of 2-Piperidone.
$^1H$-NMR($CDCl_3$) δ: 1.9-2.3(5H,m), 2.66(4H,t), 2.8(1H,t), 3.02(1H,bd), 3.33(1H,ddd), 3.92(3H,s), 3.93(3H,s), 4.91(2H,s), 4.99(2H,dd), 5.72(2H,m), 6.85(1H,d), 7.2-7.4(5H,m), 7.44(1H,d)
Specific rotation $[\alpha]_D^{25}$ = 423.39° (c = 1.0, chloroform)

Example 20 cis-(+)-4-(3,4-dimethoxyphenyl)-2-[3-(3-oxopiperazin-1-ylmethyl)benzyl]-4a,5,8,8a-tetrahydro-2H-phthalazin-1-one

The objective compound was obtained as a white amorphous substance according to the method of the step 2 in Example 10 but using 2-Piperazinone in place of Morpholine.
$^1H$-NMR($CDCl_3$) δ: 1.8-2.3(3H,m), 2.62(2H,t), 2.83(1H,t), 3.02(1H,bd), 3.13(2H,s), 3.25-3.4(3H,m), 3.54(2H,s), 3.92(3H, s), 3.93 (3H, s), 5.03(2H,dd), 5.72(2H,m), 5.89 (1H,bs), 6.86(1H,d), 7.2-7.4(5H,m), 7.44(1H,d)
Specific rotation $[\alpha]_D^{25}$ = 395.80° (c = 1.0, chloroform)

Example 21 cis-(+)-4-{4-[4-(3,4-dimethoxyphenyl)-1-oxo-4a,5,8,8a-tetrahydro-1H-phthalazin-2-ylmethyl]benzyl} morpholine 4-oxide

100 mg of cis-(+)-4-(3,4-Dimethoxyphenyl)-2-. (4-morpholin-4-ylmethylbenzyl)-4a,5,8,8a-tetrahydro-2H-pht halazin-1-one obtained in Example 2-1 was dissolved in 1 mL of MeOH and 0.062 mL of aqueous 30 % hydrogen peroxide solution was added thereto, and stirred for 24 hours at room temperature and then for 2 days at 50°C. Water was added to the reaction liquid, and the reaction liquid was subjected to extraction procedure with chloroform. This was dried with anhydrous Magnesium sulfate, and the solvent was evaporated in vacuo. The crude product was purified through silica gel column chromatography to obtain 104 mg of the objective compound as a white amorphous substance.
$^1H$-NMR($CDCl_3$) δ: 1.9-2.3(3H,m), 2.83(1H,t), 2.94(2H,d), 3.0(1H,bd), 3.2-3.4(3H,m), 3.72(2H,dd), 3.92(3H,s), 3.93(3H, s), 4.35(2H,s), 4.44(2H,dd), 5.05(2H,dd), 5.73(2H,m), 6.86(1H,d), 7.25(1H.dd), 7.4-7.5(5H,m)
Specific rotation $[\alpha]_D^{25}$ = 392.39° (c = 1.0, chloroform)

Example 22 cis-4-(4-Methoxy-3-propoxyphenyl)-2-[4-(morpholin-4-ylmethyl)benzyl]-4a,5,8,8a-tetrahydro-2H-phthalazin-1-one

Step 1 cis-4-(3-Hydroxy-4-methoxyphenyl)-2-[4-(morpholin-4-ylmethyl)benzyl]-4a,5,8,8a-tetrahydro-2H-phthalazin-1-one

After 257 mg of cis-4-(3-Cyclopentyloxy-4--methoxyphenyl)-2-[4-(morpholin-4-ylmethyl)benzyl]-4a,5,8,8a-tetrahydro-2H -phthalazin-1-one obtained in the step 2 in Example 14 was dissolved in 4 mL of toluene, 140 mg of p-Toluenesulfonic acid was added thereto and the solution was refluxed for 4 hours. After cooling, this was diluted with Ethyl acetate, and was subjected to extraction procedure. The organic layer was washed with aqueous saturated sodium hydrogencarbonate solution, water and saturated saline and then dried with anhydrous Magnesium sulfate, and the solvent was evaporated in vacuo. The crude product was purified through silica gel column chromatography to obtain 176 mg of the objective compound as a white amorphous substance.
$^1$H-NMR($CDCl_3$) δ: 1.9-2.3(3H,m), 2.42(4H,t), 2.81(1H,t), 3.01(1H,bd), 3.3(1H,ddd), 3.46(2H,s), 3.71(4H,t), 3.93(3H,s), 5.0(2H,dd), 5.71(2H,m), 6.85(1H,d), 7.3-7.5(6H,m)

Step 2 cis-4-(4-Methoxy-3-propoxyphenyl)-2-[4-(morpholin-4-ylmethyl)benzyl]-4a,5,8,8a-tetrahydro-2H-phthalazin-1-one

After 23 mg of Potassium carbonate and 22 mg of Propyl iodide were added to an anhydrous N,N-Dimethylformamide (5 mL) solution of 49 mg of cis-4-(3-Hydroxy-4-methoxyphenyl)-2-[4-(morpholin-4-ylmethyl)benzyl]-4a,5,8,8a-tetrahydro-2H -phthalazin-1-one obtained in the step 1, and the solution was stirred at room temperature for 3 hours. Water was added to the reaction liquid, and the reaction liquid was subjected to extraction procedure with Ethyl acetate. The organic layers were combined, washed with water and saturated saline and dried with Magnesium sulfate, and the solvent was evaporated in vacuo. The crude product was purified through silica gel column chromatography to obtain 29 mg of the objective compound as a white amorphous substance.
$^1$H-NMR($CDCl_3$) δ: 1.07(3H,t), 1.88(2H,dt), 1.9-2.3(3H,m), 2.42(4H,t), 2.81(1H,t), 3.02(1H,bd), 3.2(1H,ddd), 3.46(2H, s), 3.69(4H,t), 3.90(3H,s), 4.02(2H,t), 5.0(2H,dd), 5.71(2H,m), 6.85(1H,d), 7.2-7.5(6H,m)

Example 23 cis-4-(3,4-Diethoxyphenyl)-2-[4-(morpholin-4-ylmethyl)benzyl]-4a,5,8,8a-tetrahydro-2H-phthalazin-1-one

Step 1 cis-2-(4-Bromomethylbenzyl)-4-(3,4-diethoxyphenyl)-4a,5,8,8a-tetrahydro-2H-phthalazin-1-one

The objective compound was obtained as a white amorphous substance according to the method of the step 1 in Example 1 but using cis-4-(3,4-Diethoxyphenyl)-4a,5,8,8a-tetrahydro-2H-phthalazin-1-one obtained in Reference Example 7 in place of cis-4-(3,4-Dimethoxyphenyl)-4a,5,8,8a-tetrahydro-2H-phthalazin-1-one.
$^1$H-NMR($CDCl_3$) δ: 1.48(6H,t), 1.9-2.3(3H,m), 2.81(1H,t), 3.01(1H,bd), 3.32(1H,ddd), 4.13(4H,q), 4.47(2H,s), 5.01(2H, dd), 5.72(2H,m), 6.84(1H,d), 7.19-7.43(6H,m)

Step 2 cis-4-(3,4-Diethoxyphenyl)-2-[4-(morpholin-4-ylmethyl)benzyl]-4a,5,8,8a-tetrahydro-2H-phthalazin-lone

The objective compound was obtained as a white amorphous substance according to the method of the step 2 in Example 1 but using cis-2-(4-Bromomethylbenzyl)-4-(3,4-diethoxyphenyl)-4a,5,8,8a-tetrahydro-2H-phthalazin-1-one obtained in the step 1 in place of cis-2-(4-Bromomethylbenzyl)-4-(3,4-dimethoxyphenyl)-4a,5,8,8a-tetrahydro-2H-phthalazin-1-one.
$^1$H-NMR($CDCl_3$) δ: 1.47(6H,t), 1.9-2.3(3H,m), 2.42(4H,t), 2.81(1H,t), 3.03(1H,bd), 3.32(1H,ddd), 3.46(2H,s), 3.69(4H, t), 4.15(4H,q), 5.01(2H,dd), 5.71(2H,m), 6.85(1H,d), 7.2-7.4(5H,m), 7.44(1H,d)

Example 24 cis-4-(3,4-Dimethoxyphenyl)-2-(4-morpholin-4-ylbenzyl)-4a,5,8,8a-tetrahydro-2H-phthalazin-1-one

Step 1 cis-2-(4-Iodobenzyl)-4-(3,4-dimethoxyphenyl)-4a,5,8,8a-tetrahydro-2H-phthalazin-1-one

The objective compound was obtained according to the method of the step 1 in Example 1 but using 4-Iodobenzyl bromide in place of 1,4-Bis-bromomethylbenzene.

Step 2 cis-4-(3,4-Dimethoxyphenyl)-2-(4-morpholin-4-ylbenzyl)-4a,5,8,8a-tetrahydro-2H-phthalazin-1-one

After 77 mg of Copper iodide and 686 mg of Potassium phosphate were suspended in 2 mL of 2-Propanol, 0.18 mL of Ethylene glycol, 406 mg of cis-2-(4-Iodobenzyl)-4-(3,4-dimethoxyphenyl)-4a,5,8,8a-tetrahydro-2H-one-1-one obtained in the step 1 and 0.14 mL of Morpholine were added thereto in argon atmosphere, and the solution was stirred at 80˚C for 33 hours. Water was added to the reaction liquid, and the reaction liquid was subjected to extraction procedure with Ethyl acetate. The organic layers were combined, washed with water and saturated saline, then dried with Magnesium sulfate, and the solvent was evaporated in vacuo. The crude product was purified through silica gel column chromatography to obtain 41 mg of the objective compound as a white amorphous substance.
$^1$H-NMR($CDCl_3$) δ: 1.8-2.3(3H,m), 2.79(1H,t), 3.05(1H,bd), 3.12(4H,t), 3.31(1H,ddd), 3.84(4H,t), 3.92(3H,s), 3.94(3H, s), 4.95(2H,dd), 5.70(2H,m), 6.8-6.9(3H,m), 7.2-7.3(3H,m), 7.46(1H,d)

Example 25 cis-4-(3-Ethoxy-4-methoxyphenyl)-2-[4-(morpholin-4-ylmethyl)benzyl]-4a,5,8,8a-tetrahydro-2H-phthalazin-1-one

The objective compound was obtained according to the method of the step 2 in Example 22 but using Ethyl iodide in place of Propyl iodide.
$^1$H-NMR($CDCl_3$) δ: 1.49(3H,t), 1.9-2.3(3H,m), 2.42(4H,t), 2.81(1H,t), 3.02(1H,bd), 3.2(1H,ddd), 3.46(2H,s), 3.69(4H,t), 3.91(3H,s), 4.14(2H,q), 5.0(2H,dd), 5.71(2H,m), 6.85(1H,d), 7.2-7.5(6H,m)

Example 26 cis-4-(3-Isopropoxy-4-methoxyphenyl)-2-[4-(morpholin-4-ylmethyl)benzyl]-4a,5,8,8a-tetrahydro-2H-phthalazin-1-one

The objective compound was obtained according to the method of the step 2 in Example 22 but using Isopropyl iodide in place of Propyl iodide.
$^1$H-NMR($CDCl_3$) δ: 1.39(6H,d), 1.9-2.3(3H,m), 2.42(4H,t), 2.81(1H,t), 3.02(1H,bd), 3.2(1H,ddd), 3.46(2H,s), 3.69(4H,t), 3.89(3H,s), 4.58(1H,tt), 5.0(2H,dd), 5.71(2H,m), 6.88(1H,d), 7.2-7.5(6H,m)
The structural formulae of the compounds of the invention obtained in the above Examples 1 to 26 are shown in Table 1.

[Table 1-1]

| Example No. | Structural Formula | Example No. | Structural Formula |
|---|---|---|---|
| 1 | 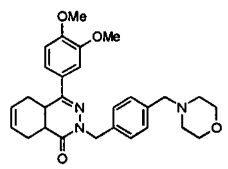 4a,8a-cis | 7 | 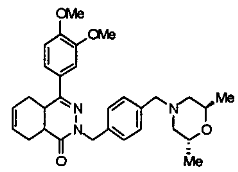 4a,8a-cis |
| 2-1 | 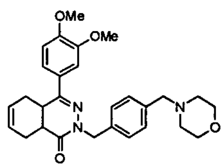 4a,8a-cis-(+) | 8 | 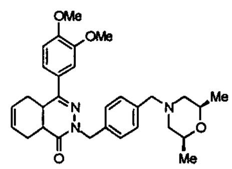 4a,8a-cis |
| 2-2 | 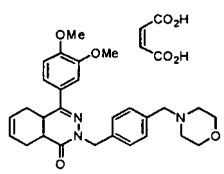 4a,8a-cis-(+) | 9 | 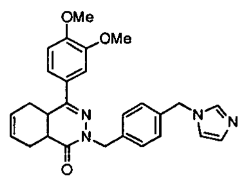 4a,8a-cis |
| 3 | 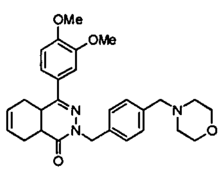 4a,8a-cis-(-) | 10 | 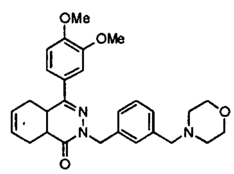 4a,8a-cis |

(continued)

| Example No. | Structural Formula | Example No. | Structural Formula |
|---|---|---|---|
| 4 | 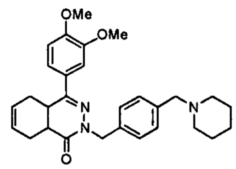 4a,8a-cis | 11 | 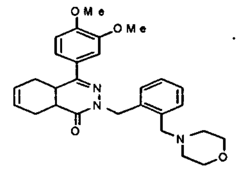 4a,8a-cis |
| 5 | 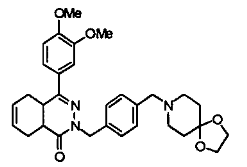 4a,8a-cis | 12 | 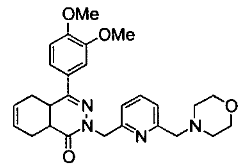 4a,8a-cis |
| 6 | 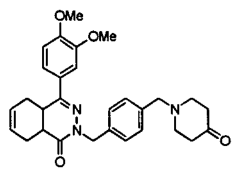 4a,8a-cis | 13 | 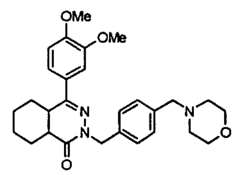 4a,8a-cis |

[Table 1-2]

| Example No. | Structural Formula | Example No. | Structural Formula |
|---|---|---|---|
| 14 | 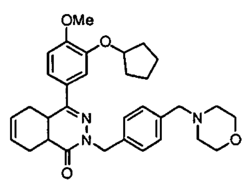 4a,8a-cis | 21 | 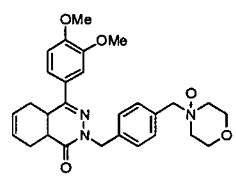 4a,8a-cis-(+) |
| 15 | 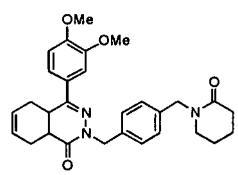 4a,8a-cis-(+) | 22 | 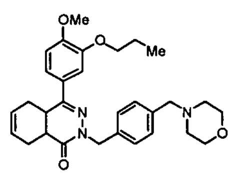 4a,8a-cis |
| 16 | 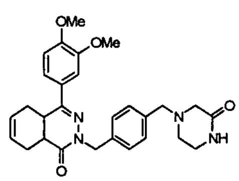 4a,8a-cis-(+) | 23 | 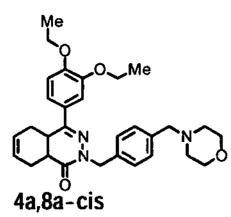 4a,8a-cis |

(continued)

| Example No. | Structural Formula | Example No. | Structural Formula |
|---|---|---|---|
| 17 | 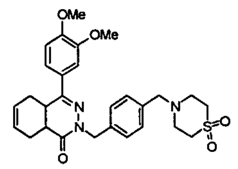 4a,8a-cis-(+) | 24 | 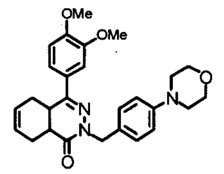 4a,8a-cis |
| 18 | 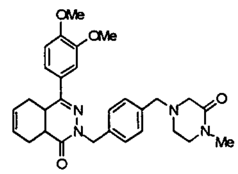 4a,8a-cis-(+) | 25 | 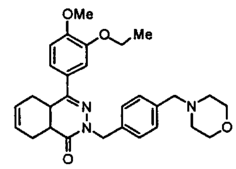 4a,8a-cis |
| 19 | 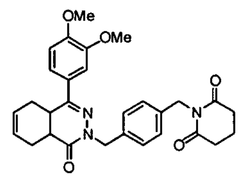 4a,8a-cis-(+) | 26 | 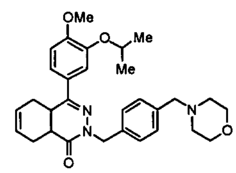 4a,8a-cis |
| 20 | 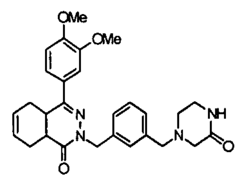 4a,8a-cis-(+) | | |

Test Example 1 - PDE4 Inhibitory Activity

The activity of a test compound to inhibit human PDE4 was evaluated in the manner mentioned below.
In the presence of a test compound ($10^{-7}$ to $10^{-12}$ M), 1 pmol of cAMP was hydrolyzed through incubation with 5 ng of human recombinant PDE4D (ABNOVA's #H00005144-P01, Lot. FAK0050713Jb01, Taiwan) at 30˚C for 30 minutes. After the hydrolysis, the cAMP concentration was measured with a chemiluminescent kit, HitHunter cAMP II Assay Kit (by Amersham Bioscience). The concentration of a test compound and the relative light unit (RLU) within the region where the test compound inhibited PDE4 were exponentially approximated; and the concentration at a point where the regression curve crosses RLU obtained by a hydrolysis with a half amount (2.5 ng) of the enzyme under the same condition, as measured at the same time, is defined as $IC_{50}$. The result is shown in Table 2.

[Table 2]

| Test Compound | PDE4 inhibitory activity ($IC_{50}$:μM) |
|---|---|
| Example 1 | 0.063 |
| Example 2-2 | 0.00079 |
| Example 5 | 0.020 |
| Example 6 | 0.0013 |
| Example 9 | 0.025 |
| Example 10 | 0.020 |

(continued)

| Test Compound | PDE4 inhibitory activity ($IC_{50}$:μM) |
|---|---|
| Example 13 | 0.0050 |

As shown in Table 2, it is obvious that the compounds of the invention have an excellent PDE4 inhibitory activity.

Test Example 2 - TNF-α Production-Suppressing Activity

The activity of a test compound to suppress TNF-α production from rat whole blood cells stimulated with LPS was evaluated in the manner mentioned below (J. Pharmacol. Exp. Ther., 302, 390-396, 2000).
Under anesthesia with ether, a proper amount of blood was taken out from a 10-week male SD rat (♂, 10-week old) through its abdominal vein into a tube with EDTA (1.6 mg/mL) therein. A test compound was dissolved in dimethyl sulfoxide to prepare its $10^{-2}$ M solution, which was diluted before use. 80 μL of rat blood was dispensed into each well of a 96-well plate, then 10 μL of the test compound solution was added thereto, and incubated at 37˚C in 5% $CO_2$ for 30 minutes. The test compound and the blood were well mixed, then 10 μL of LPS (100 μg/mL) was added thereto, and incubated at 37˚C in 5% $CO_2$ for 5 hours. Further, 100 μL of PBS was added to each well, then the plate was centrifuged at 4000 rpm for 2 minutes to collect the supernatant, and this was frozen and stored at -80˚C until its use for analysis. The amount of TNF-α production was measured through ELISA. $IC_{50}$ for TNF-α production-suppressing activity was calculated as the test compound concentration for 50 % inhibition of TNF-α production.
The result is shown in Table 3. As a control compound, used was the above-mentioned known compound (compound of general formula [A] in which R is a carboxyl group, cis-4-[4-(3,4-dimethoxyphenyl)-1-oxo-4a,5,8,8a-tetrahydro-1H-phthalazin-2-yl-methyl]benzoic acid (hereinafter referred to as control compound A)).

[Table 3]

| Test Compound | TNF-α Production-Suppressing Activity ($IC_{50}$:μM) |
|---|---|
| Example 1 | 0.0043 |
| Example 2-1 | 0.0024 |
| Example 2-2 | 0.0011 |
| Example 5 | 0.199 |
| Example 6 | 0.084 |
| Example 9 | 0.001 |
| Example 10 | 0.107 |
| Example 13 | 0.059 |
| Example 16 | 0.0049 |
| Example 20 | 0.019 |
| Example 21 | 0.012 |
| Example 24 | 0.012 |
| control compound A | 133.181 |

As shown in Table 3, it is obvious that the compounds of the invention have an extremely strong TNF-α production-suppressing effect as compared with the control compound A.

Test Example 3 - Dermatitis Inhibitory Effect in a Dermatitis Model

The dermatitis inhibitory effect of a test compound was investigated, using a trinitrochlorobenzene (TNCB)-induced dermatitis model (J. Invest. Dermatol., 105, 749-755, 1995, J. Immuno., 159, 2484-2491, 1997).
20 μL of 1 % (w/v) TNCB/acetone solution was applied dropwise to both sides of the right pinna of a BALB/C mouse (♂, 8-week old), whereby the mouse was sensitized. From day 7 after the sensitization with TNCB, 1 % TNCB/acetone solution was applied to both sides of the right pinna of the mouse every other day at 3 times/week, for 3 weeks in total. TNCB was applied for 2 weeks so as to fully induce dermatitis in the mouse, and then 20 mg of 1.0 % test compound/vaseline ointment was applied to both sides of the right pinna five times in total for 1 week. At 6 hours after the last application, the thickness of the right pinna of the mouse was measured, and the effect of the test compound on dermatitis was thus evaluated. The inhibitory rate was calculated according to the following formula. The result is shown in Table 4.

Inhibitory Rate (%)

= {1-[mean thickness of inflamed pinna (right ear) of chemical-treated group - mean thickness of non-inflamed pinna (left ear) of the group]/[mean thickness of inflamed pinna (right ear) of diseased control group - mean thickness of non-inflamed pinna (left ear) of the group]} × 100.

[Table 4]

| Test Compound | Dermatitis Inhibitory Percentage (%) |
|---|---|
| Example 1 | 31.2 |
| Example 2-1 | 38.5 |
| Example 5 | 20.2 |
| Example 6 | 36.8 |
| Example 10 | 31.1 |
| Example 13 | 33.2 |
| control compound A | 2.6 |

As obvious from Table 4, also in the in-vivo test system, the compounds of the invention of Examples have a remarkable therapeutical effect on TNCB-induced dermatitis as compared with the control compound A.

Test Example 4 - Metabolism by Human Liver Microsome

The metabolic characteristics of a test compound were investigated, using human liver microsome.
400 μL of EP buffer (0.25 mM EDTA, 0.25 M potassium phosphate buffer, pH 7.4), 50 μL of 20 mg/mL human liver microsome fraction (by KAC), 350 μl of pure water and 100 μL of 1 × $10^{-4}$ M test compound were put into a 1.5 mL microtube, and preincubated at 37°C for 5 minutes. 100 μL of an NADPH producing system (containing 20 mg of NADP+, 70 mg of G6P, 40 units of G6PDH, 20 mg of magnesium chloride hexahydrate in 1 mL of pure water) was added to it, and incubated. To 100 μL of the sample taken at 10 minutes after the start of the incubation, added was the same amount of acetonitrile, and the reaction was thereby stopped. This was centrifuged at 10, 000 rpm for 5 minutes, and then the supernatant was recovered. 20 μL of the supernatant was led into a high-performance liquid chromatography (Shimadzu's SPD-10A, LC-10AS, CTO-10A, C-R6A), and the peak area of the test compound at 320 nm was measured, using a C18 column (Cadenza, CD-C18, 75 x 4.6 mm). For the compound obtained in Example 2-1, the mobile phase for measurement was an aqueous solution of 30 % acetonitrile/0.1 % methanesulfonic acid. For the control compound A, the mobile phase was an aqueous solution of 50 % acetonitrile/0.1 % methanesulfonic acid. Under the condition, the peak area of the test compound was measured. The metabolic rate was calculated as follows: 100 μL of acetonitrile was added to 90 μL of the above pre-incubated solution, and then 10 μl of the NADPH producing system was added thereto, and the resulting solution was analyzed for the peak area of the test compound. Based on it, the metabolic rate in 10 minutes after the incubation was calculated. The result is shown in Table 5.

[Table 5]

| Test Compound | Metabolic Rate (%) |
|---|---|
| Example 2-1 | 61.5 |
| control compound A | 35.2 |

As shown in Table 5, the compound obtained in Example 2-1 is more rapidly metabolized than the control compound A. Accordingly, when the compound of the invention is used as an external-use preventive/therapeutical agent for inflammatory or autoimmune diseases, the reduction in its side effects on central nervous systems (nausea, emesis, headache), intrinsic to PDE4 inhibitor, may be expected (for example, see Non-Patent References 74, 76 to 80).

Test Example 5 - Effect in Combination with Steroid

The effect of a test compound in combination with a steroid was investigated, using a 4-ethoxymethylene-2-phenyl-2-oxazolin-5-one (oxazolone)-induced dermatitis model (Pharmacol., 75, 45-52, 2005, Br. J. Dermatolol., 151, 1133-1142, 2004).

The antigen sensitization was attained by applying dropwise 20 μL of 1 % (w/v) oxazolone/acetone solution to both sides of the right pinna of a Balb/c mouse (♂, 8-week old). From the 7th day after the antigen sensitization (this is day 0), dermatitis was induced by applying dropwise the solution to both sides of the right pinna of the mouse, on days 2, 4, 7, 9, 11, 14, 16, 18, 21, 23, 25, 28 and 30. After the dermatitis was fully induced, the effect of a test compound was investigated. A test compound-containing ointment was applied to the mouse on days 14 to 18, days 21 to 25, and days 28 to 30. In this test, the following three ointments were used.

1) Vaseline ointment containing 1 % of the compound obtained in Example 2-2 (ointment A).
2) Commercial product, Rinderon® V ointment (by Shionogi) (containing 0.12 % betamethasone valerate, ointment B).
3) Commercial product, Rinderon® V ointment (by Shionogi) diluted with vaseline to 1/64 (v/v) (ointment C).

25 μL of the ointment was applied to the right pinna of the mouse. At 6 hours after the oxazolone application on day 30, the thickness of the right pinna of the mouse was measured, and the effect of the test compound on dermatitis was thereby evaluated. The inhibitory rate was calculated according to the following formula. The result is shown in Table 6.

Inhibitory Rate (%) = {1-[mean thickness of inflamed pinna (right ear) of chemical-treated group - mean thickness of pinna (right ear) of normal group]/[mean thickness of inflamed pinna (right ear) of diseased control group - mean thickness of pinna (right ear) of normal group]} × 100.

[Table 6]

| Ointment | Inhibitory Percentage (%) |
|---|---|
| Ointment A | 56.0 |
| Ointment B | 73.0 |
| Ointment C | 20.2 |
| Ointment A+ Ointment C | 71.9 |

As shown in Table 6, when the case of single use of the commercial steroid (ointment B) is compared with the case of combination of the ointment containing a compound of the invention (ointment A) and the commercial steroid, of which the dose was reduced to 1/64, .(ointment C), the two are the same in their therapeutic effect on dermatitis. The therapeutical effect in the combination of the ointments A and C is much superior to that expected in the single use of the individual ointments.

Test Example 6 - Sequential Therapy with Steroid

Sequential therapy with a test compound and a steroid was investigated, using a 4-ethoxymethylene-2-phenyl-2-oxazolin-5-one (oxazolone)-induced dermatitis model (Pharmacol., 75, 45-52, 2005, Br. J. Dermatolol., 151, 1133-1142, 2004).
The antigen sensitization was attained by applying dropwise 20 μL of 1 % (w/v) oxazolone/acetone solution to both sides of the right pinna of a Balb/c mouse (♂, 8-week old). From the 7th day after the antigen sensitization (this is day 0), dermatitis was induced by applying dropwise the solution to both sides of the right pinna of the mouse, on days 2, 4, 7, 9, 11, 14, 16, 18, 21, 23, 25, 28, 30, 32, 35, 37, 39. Rinderon® V ointment (by Shionogi) was applied on days 14 to 18 (for 5 days). Afterwards, the mice were divided into a group to which Rinderon V is continued to be applied, a group to which vaseline ointment containing 1 % of the compound obtained in Example 2-2 is applied, and a group to which vaseline alone is applied. The chemical was applied to the mice of those groups on days 21 to 25, days 28 to 32, and days 35 to 39. At 6 hours after the oxazolone application on day 0, 11, 18, 25, 32 or 39, the thickness of the right and left pinnae of the mouse was measured, and the thickness difference between the pinnae was defined as ear swelling, which indicates the effect of the test compound on dermatitis. The ear swelling rate was calculated according to the following formula. The result is shown in Fig. 1.

Ear Swelling Rate (%)
= (thickness of inflamed pinna (right ear) of chemical-treated group - mean thickness of non-inflamed pinna (left ear) of the group/(thickness of the inflamed pinna (right ear) of the diseased control group - mean thickness of non-inflamed pinna (left ear) of the group) × 100.

As shown in Fig. 1, when the steroid was replaced by the ointment containing a compound of the invention after its continuous application for 5 days, the therapeutical effect in the case was equal to the other case where the steroid application was continued after that. Accordingly, the compound of the invention may be applicable to the treatment for dermatitis for which steroid is used at present, and therefore it may evade long-term application of steroid that needs special care in its use. The compound of the invention may be expected as a drug capable of providing a novel therapeutical method.
Preparation Example 1:

Tablet (for oral application):

In one tablet of 85 mg,

| | |
|---|---|
| Compound of Example 2-1 | 10.0 mg, |
| Corn starch | 46.6 mg, |
| Crystalline cellulose | 24.0 mg, |
| Methyl cellulose | 4.0 mg, |
| Magnesium stearate | 0.4 mg. |

A mixed powder in the above ratio is tabletted in an ordinary manner into oral tablets.

Preparation Example 2:

Ointment (for external application):

In ointment of 1 g,

| | |
|---|---|
| Compound of Example 2-2 | 3.0 mg |
| White vaseline | 997.0 mg |

After white vaseline is emulsified, the compound of Example 2-2 is mixed with it and totally homogenized to be an ointment.

[Industrial Applicability]

The compound of the invention or its pharmaceutically acceptable salt exhibits a strong PDE4 inhibitory activity as its effect and mechanism and especially has an excellent activity to suppress production of TNF-$\alpha$ that is said to have a strongest inflammatory-inducing capability. Accordingly, the compound of the invention or its pharmaceutically acceptable salt are useful as a preventive/therapeutical agent for diseases correlated with various inflammatory cytokines such as typically TNF-$\alpha$, for example, many inflammatory or autoimmune diseases such as atopic dermatitis, contact dermatitis, psoriasis, allergic rhinitis, allergic conjunctivitis, chronic rheumatism, ulcerative colitis, Crohn's disease, sepsis, toxic shock syndrome, nephritis, hepatitis, circulatory insufficiency (cardiac insufficiency, arteriosclerosis, cerebral stroke), multiple sclerosis, AIDS, allograft rejection, osteoarthritis, autoimmune diabetes, and autoimmune encephalomyelitis. In particular, they are useful as a preventive/therapeutical agent for dermatitic diseases (atopic dermatitis, contact dermatitis, psoriasis). In addition, they are useful as a preventive/therapeutical agent for other diseases that are known to be correlated with PDE4, for example, asthma, chronic bronchitis, chronic obstructive pulmonary disease (COPD), pneumonic diseases, and adult respiratory distress syndrome (ARDS).

## Claims

**1.** A phthalazinone derivative of the following general structural formula [1] or a pharmaceutically acceptable salt thereof:

[Chemical Formula 1]

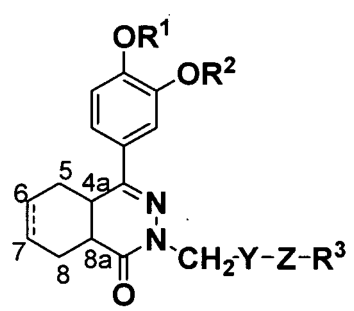

[1]

in the structure formula [1],
the following partial structure bridging the 6-position and the 7-position represents a single bond or a double bond:

[Chemical Formula 2]

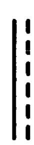

$R^1$ and $R^2$ are the same or different and each represents alkyl or cycloalkyl,
Y represents phenylene, or a divalent aromatic heterocyclic group which is made up of 6 ring-constituting atoms and has 1 or 2 nitrogen atoms as the ring-constituting atoms,
Z represents a single bond, alkylene, alkenylene or alkynylene,
$R^3$ represents a mono- to tri-cyclic saturated or unsaturated cyclic amino group which is made up of 5 to 10 ring-constituting atoms and has at least one nitrogen atom, and besides, optionally 1 to 3 nitrogen, oxygen or sulfur atoms as the ring-constituting atoms; the cyclic amino group may be substituted with $R^{31}$ and $R^{32}$, in which the nitrogen atom as the ring-constituting atom may form an oxide,
$R^{31}$ and $R^{32}$ are the same or different and each represents alkyl, alkoxy, hydroxy, halogen or oxo; or $R^{31}$ and $R^{32}$ are taken together to form methylenedioxy or ethylenedioxy.

**2.** The phthalazinone derivative or the pharmaceutically acceptable salt thereof as claimed in claim 1, which has a 4a, 5,8,8a-tetrahydro-2H-phthalazin-1-one skeleton.

**3.** The phthalazinone derivative or the pharmaceutically acceptable salt thereof as claimed in any of claim 1 or 2, wherein $R^1$ and $R^2$ are the same or different and each represents alkyl.

**4.** The phthalazinone derivative or the pharmaceutically acceptable salt thereof as claimed in any of claim 1 or 2, wherein Y represents phenylene.

**5.** The phthalazinone derivative or the pharmaceutically acceptable salt thereof as claimed in any of claim 1 or 2, wherein Z represents a single bond or alkylene.

**6.** The phthalazinone derivative or the pharmaceutically acceptable salt thereof as claimed in any of claim 1 or 2, wherein Z represents alkylene.

**7.** The phthalazinone derivative or the pharmaceutically acceptable salt thereof as claimed in any of claim 1 or 2, wherein $R^3$ represents morpholin-4-yl, 4-oxido-morpholin-4-yl, piperidin-1-yl, piperazin-1-yl, thiomorpholin-4-yl or imidazol-1-yl optionally substituted with $R^{31}$ and $R^{32}$, and $R^{31}$ and $R^{32}$ are the same or different and each represents alkyl or oxo.

**8.** The phthalazinone derivative or the pharmaceutically acceptable salt thereof as claimed in claim 1, which has a 4a, 5,8,8a-tetrahydro-2H-phthalazin-1-one skeleton and wherein $R^1$ and $R^2$ are the same or different and each represents alkyl, Y represents phenylene, Z represents a single bond or alkylene, $R^3$ represents morpholin-4-yl, 4-oxido-morpholin-4-yl, piperidin-1-yl, piperazin-1-yl, thiomorpholin-4-yl or imidazol-1-yl optionally substituted with $R^{31}$ and $R^{32}$, and $R^{31}$ and $R^{32}$ are the same or different and each represents alkyl or oxo.

**9.** The phthalazinone derivative or the pharmaceutically acceptable salt thereof as claimed in claim 1, which has a 4a, 5,8,8a-tetrahydro-2H-phthalazin-1-one skeleton and wherein $R^1$ and $R^2$ are the same or different and each represents alkyl, Y represents phenylene, Z represents alkylene, $R^3$ represents morpholin-4-yl, 4-oxido-morpholin-4-yl, piperidin-1-yl, piperazin-1-yl, thiomorpholin-4-yl or imidazol-1-yl optionally substituted with $R^{31}$ and $R^{32}$, and $R^{31}$ and $R^{32}$ are the same or different and each represents alkyl or oxo.

**10.** The phthalazinone derivative or the pharmaceutically acceptable salt thereof as claimed in claim 1, which is any of the following (1) to (26):

(1) cis-4-(3,4-Dimethoxyphenyl)-2-[4-(morpholin-4-ylmethyl)benzyl]-4a,5,8,8a-tetrahydro-2H-phthalazin-1-one
(2) cis-(+)-4-(3,4-Dimethoxyphenyl)-2-[4-(morpholin-4-ylmethyl)benzyl]-4a,5,8,8a-tetrahydro-2H-phthalazin-1-one
(3) cis-(-)-4-(3,4-Dimethoxyphenyl)-2-[4-(morpholin-4-ylmethyl)benzyl]-4a,5,8,8a-tetrahydro-2H-phthalazin-1-one
(4) cis-4-(3,4-Dimethoxyphenyl)-2-[4-(piperidin-1-ylmethyl)benzyl]-4a,5,8,8a-tetrahydro-2H-phthalazin-1-one
(5) cis-4-(3,4-Dimethoxyphenyl)-2-[4-(1,4-dioxa-8-azaspiro[4,5]dec-8-ylmethyl)benzyl]-4a,5,8,8a-tetrahydro-2H-phthalazin-1-one
(6) cis-4-(3,4-Dimethoxyphenyl)-2-[4-(4-oxopiperidin-1-ylmethyl)benzyl]-4a,5,8,8a-tetrahydro-2H-phthalazin-1-one
(7) cis-4-(3,4-Dimethoxyphenyl)-2-[4-(trans-2,6-dimethylmorpholin-4-ylmethyl)benzyl]-4a,5,8,8a-tetrahydro-2H-phthalazin-1-one

(8) cis-4-(3,4-Dimethoxyphenyl)-2-[4-(cis-2,6-dimethylmorpholin-4-ylmethyl)benzyl]-4a,5,8,8a-tetrahydro-2H-phthalazin-1-one
(9) cis-4-(3,4-Dimethoxyphenyl)-2-[4-(imidazol-1-ylmethyl)benzyl]-4a,5,8,8a-tetrahydro-2H-phthalazin-1-one
(10) cis-4-(3,4-Dimethoxyphenyl)-2-[3-(morpholin-4-ylmethyl)benzyl]-4a,5,8,8a-tetrahydro-2H-phthalazin-1-one
(11) cis-4-(3,4-Dimethoxyphenyl)-2-[2-(morpholin-4-ylmethyl)benzyl]-4a,5,8,8a-tetrahydro-2H-phthalazin-1-one
(12) cis-4-(3,4-Dimethoxyphenyl)-2-[6-(morpholin-4-ylmethyl) pyridin-2-ylmethyl]-4a, 5,8,8a-tetrahydro-2H-phthalazin-1-one
(13) cis-4-(3,4-Dimethoxyphenyl)-2-[4-(morpholin-4-ylmethyl)benzyl]-4a,5,6,7,8,8a-hexahydro-2H-phthalazin-1-one
(14) cis-4-(3-Cyclopentyloxy-4-methoxyphenyl)-2-[4-(morpholin-4-ylmethyl)benzyl]-4a,5,8,8a-tetrahydro-2H-phthalazin-1-one
(15) cis-(+)-4-(3,4-Dimethoxyphenyl)-2-[4-(2-oxo-piperidin-1-ylmethyl) benzyl]-4a, 5,8,8a-tetrahydro-2H-phthalazin-1-one
(16) cis-(+)-4-(3,4-Dimethoxyphenyl)-2-[4-(3-oxopiperazin-1-ylmethyl) benzyl]-4a, 5,8,8a-tetrahydro-2H-phthalazin-1-one
(17) cis-(+)-4-(3,4-Dimethoxyphenyl)-2-[4-(1,1-dioxidothiomorpholin-4-ylmethyl)benzyl]-4a,5,8,8a-tetrahydro-2H-phthalazin-1-one
(18) cis-(+)-4-(3,4-Dimethoxyphenyl)-2-[4-(4-methyl-3-oxopiperazin-1-ylmethyl)benzyl]-4a,5,8,8a-tetrahydro-2H-phthalazin-1-one
(19) cis-(+)-4-(3,4-Dimethoxyphenyl)-2-[4-(2,6-dioxo-piperidin-1-ylmethyl) benzyl]-4a, 5,8,8a-tetrahydro-2H-phthalazin-1-one
(20) cis-(+)-4-(3,4-Dimethoxyphenyl)-2-[3-(3-oxopiperazin-1-ylmethyl) benzyl]-4a, 5,8,8a-tetrahydro-2H-phthalazin-1-one
(21) cis-(+)-4-{4-[4-(3,4-Dimethoxyphenyl)=1-oxo-4a,5,8,8a-tetrahydro-1H-phthalazin-2-ylmethyl]benzyl} morpholine 4-oxide
(22) cis-4-(4-Methoxy-3-propoxyphenyl)-2-[4-(morpholin-4-ylmethyl) benzyl]-4a, 5,8,8a-tetrahydro-2H-phthalazin-1-one
(23) cis-4-(3,4-Diethoxyphenyl)-2-[4-(morpholin-4-ylmethyl)benzyl]-4a,5,8,8a-tetrahydro-2H-phthalazin-1-one
(24) cis-4-(3,4-Dimethoxyphenyl)-2-(4-morpholin-4-ylbenzyl)-4a,5,8,8a-tetrahydro-2H-phthalazin-1-one
(25) cis-4-(3-Ethoxy-4-methoxyphenyl)-2-[4-(morpholin-4-ylmethyl) benzyl]-4a, 5,8,8a-tetrahydro-2H-phthalazin-1-one
(26) cis-4-(3-Isopropoxy-4-methoxyphenyl)-2-[4-(morpholin-4-ylmethyl) benzyl]-4a, 5,8,8a-tetrahydro-2H-phthalazin-1-one

**11.** A pharmaceutical composition comprising as the active ingredient a phthalazinone derivative or a pharmaceutically acceptable salt thereof as claimed in any of claims 1 to 9.

**12.** A type 4 phosphodiesterase inhibitor comprising as the active ingredient a phthalazinone derivative or a pharmaceutically acceptable salt thereof as claimed in any of claims 1 to 9.

**13.** A TNF-α production suppressor comprising as the active ingredient a phthalazinone derivative or a pharmaceutically acceptable salt thereof as claimed in any of claims 1 to 9.

**14.** A preventive or therapeutical agent for atopic dermatitis, contact dermatitis, psoriasis, allergic rhinitis, allergic conjunctivitis, ulcerative colitis, Crohn's disease, asthma or chronic obstructive pulmonary disease, which comprises as the active ingredient a phthalazinone derivative or a pharmaceutically acceptable salt thereof as claimed in any of claims 1 to 9.

**15.** An external preparation for prevention or treatment of atopic dermatitis, contact dermatitis, psoriasis, allergic rhinitis, allergic conjunctivitis, ulcerative colitis, Crohn's disease, asthma or chronic obstructive pulmonary disease, which comprises as the active ingredient a phthalazinone derivative or a pharmaceutically acceptable salt thereof as claimed in any of claims 1 to 9.

[Fig.1]

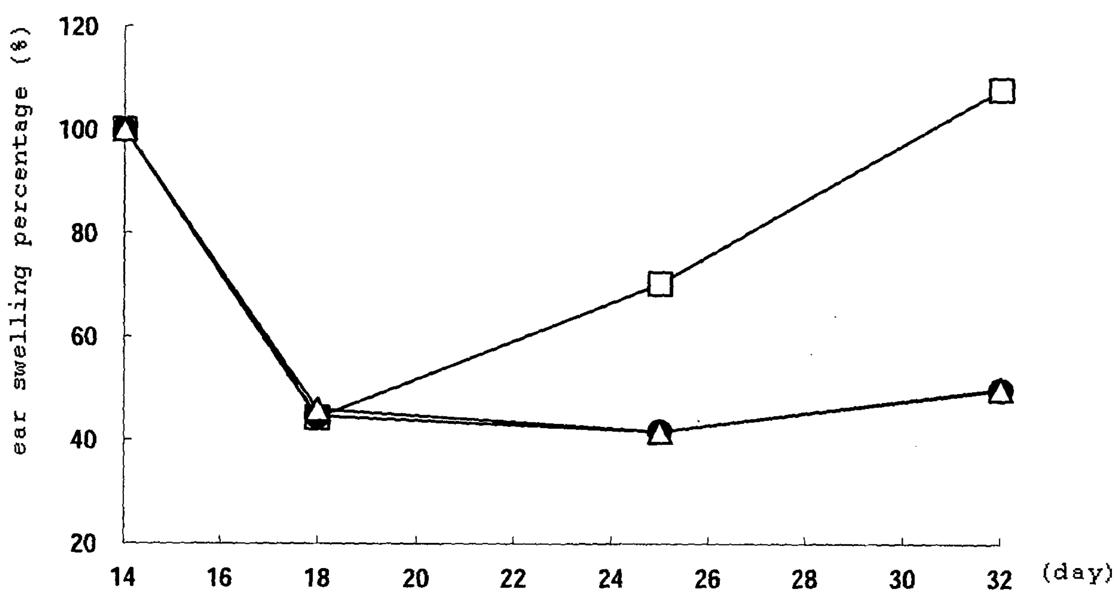
ear swelling percentage (%)
120
100
80
60
40
20
14
16
18
20
22
24
26
28
30
32
(day)

**INTERNATIONAL SEARCH REPORT**

International application No.
PCT/JP2006/308460

A. CLASSIFICATION OF SUBJECT MATTER
***C07D237/32***(2006.01), ***A61K31/502***(2006.01), ***A61K31/5377***(2006.01), ***A61P1/04***(2006.01), ***A61P11/00***(2006.01), ***A61P11/02***(2006.01), ***A61P11/06***(2006.01), ***A61P17/00***(2006.01), ***A61P17/06***(2006.01), ***A61P27/14***(2006.01),
According to International Patent Classification (IPC) or to both national classification and IPC

B. FIELDS SEARCHED

Minimum documentation searched (classification system followed by classification symbols)
A61K31/502, A61K31/5377, A61P1/04, A61P11/00, A61P11/02, A61P11/06, A61P17/00, A61P17/06, A61P27/14, A61P37/08, A61P43/00, C07D237/32, C07D401/10, C07D403/10, C07D413/10, C07D491/113

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| A | VAN DER MEY, M. et al., Novel Selective PDE4 Inhibitors. 3. In Vivo Antiinflammatory Activity of a New Series of N-Substituted cis-Tetra- and cis-Hexahydrophthalazinones, Journal of Medicinal Chemistry, 2002, Vol.45, No.12, pages 2520 to 2525 | 1-15 |
| A | VAN DER MEY, M. et al., Novel Selective PDE4 Inhibitors. 1. Synthesis, Structure-Activity Relationships, and Molecular Modeling of 4-(3,4-Dimethoxyphenyl)-2H-phthalazin-1-ones and Analogues, Journal of Medicinal Chemistry, 2001, Vol.44, No.16, pages 2511 to 2522 | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>22 May, 2006 (22.05.06) | Date of mailing of the international search report<br>30 May, 2006 (30.05.06) |
| Name and mailing address of the ISA/<br>Japanese Patent Office<br><br>Facsimile No. | Authorized officer<br><br>Telephone No. |

Form PCT/ISA/210 (second sheet) (April 2005)

**INTERNATIONAL SEARCH REPORT**

International application No.
PCT/JP2006/308460

C (Continuation). DOCUMENTS CONSIDERED TO BE RELEVANT

| Category* | Citation of document, with indication, where appropriate, of the relevant passages | Relevant to claim No. |
|---|---|---|
| A | VAN DER MEY, M. et al., Novel Selective PDE4 Inhibitors. 2. Synthesis and Structure-Activity Relationships of 4-Aryl-Substituted cis-Tetra- and cis-Hexahydrophthalazinones, Journal of Medicinal Chemistry, 2001, Vol.44, No.16, pages 2523 to 2535 | 1-15 |
| A | WO 98/31674 A1 (BYK GULDEN LOMBERG CHEMISCHE FABRIK GMBH), 23 July, 1998 (23.07.98), Full text & EP 971901 A1 & JP 2001-508078 A & US 6103718 A | 1-15 |
| A | JP 2004-526785 A (Altana Pharma AG.), 02 September, 2004 (02.09.04), Full text & WO 02/85885 A1 & EP 1385838 A1 & US 2004/132721 A1 | 1-15 |
| A | JP 2004-526789 A (Altana Pharma AG.), 02 September, 2004 (02.09.04), Full text & WO 02/85906 A2 & EP 1385848 A2 & US 2004/127707 A1 | 1-15 |
| A | JP 2003-512459 A (Altana Pharma AG.), 02 April, 2003 (02.04.03), Full text & WO 01/30766 A1 & EP 1228046 A1 & US 6756371 B1 | 1-15 |

Form PCT/ISA/210 (continuation of second sheet) (April 2005)

**INTERNATIONAL SEARCH REPORT**

International application No.
PCT/JP2006/308460

Continuation of A. CLASSIFICATION OF SUBJECT MATTER
(International Patent Classification (IPC))

***A61P37/08***(2006.01), ***A61P43/00***(2006.01), ***C07D401/10***(2006.01), ***C07D403/10***(2006.01), ***C07D413/10***(2006.01), ***C07D491/113***(2006.01)

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9831674 A


### Non-patent literature cited in the description


- *Biochem.Biophys.Acta,* 1994, vol. 1224, 467-479
- *Trends Biochem.Sci,* 1997, vol. 22, 217-224
- *Nat.Rev.Mol.Cell Biol,* 2002, vol. 3, 710-718
- *Front Neuroendocrinol,* 2000, vol. 21, 103-132
- *Curr.Opin.Cell Biol,* 2000, vol. 12, 174-179
- *Mol.Pharmacol.,* 1994, vol. 46, 399-405
- *Drugs,* 2000, vol. 59, 193-212
- *J.Bio.Chem,* 2003, vol. 278, 5493-5496
- *Clin.Exp.Allergy,* 1993, vol. 22, 337-344
- *Biochem.Pharmacol,* 1999, vol. 57, 965-973
- *Eur.Respir.J,* 1995, vol. 8, 1179-83
- *Pharmaco.Ther,* 1991, vol. 51, 13-34
- *Am.J.Respir.Crit.Care Med,* 1998, vol. 157, 351-370
- *JPET,* 2001, vol. 297, 267-279
- *Biochem.J.,* 2003, vol. 370, 1-18
- *Br.J.Pharmacol,* 1998, vol. 123, 631-636
- *Br.J.Pharmacol,* 1998, vol. 124, 547-555
- *J.Allergy Clin.Immunol,* 1999, vol. 103, S421-S428
- *Int.Arch.Allergy Immunol,* 1997, vol. 114, 348-353
- *JPET,* 1996, vol. 278, 1356-1361
- *Eur.J.Pharmacol.,* 1999, vol. 367, 343-350
- *J.Allergy Clin.Immunol.,* 1997, vol. 99, 28-37
- *PNAS,* 1975, vol. 72, 3666-3670
- *Science,* 1985, vol. 230, 630-632
- *Nat.Rev.Immunol,* 2003, vol. 3, 745-756
- *Cell,* 2001, vol. 104, 487-501
- *Science,* 1986, vol. 234, 470-474
- *Chem.Immunol,* 2000, vol. 74, 141-161
- *Eur.Cytokine Netw,* 1996, vol. 7, 93-124
- *Nature,* 1997, vol. 385, 733-736
- *Microsc.Res.Tech,* 2000, vol. 50, 184-195
- *Lancet,* 1989, vol. 2, 244-247
- *Trends Cell Biol,* 2001, vol. 11, 372-377
- *Nat.Cell Biol,* 2002, vol. 4, 131-136
- *PNAS,* 1999, vol. 96, 3763-3768
- *EMBO.J.,* 1996, vol. 15, 1914-1923
- *J.Bio.Chem,* 1998, vol. 273, 3285-3290
- *Eur.Cytokine Netw,* 1995, vol. 6, 225-230
- *Science,* 1999, vol. 285, 248-251
- *J.Immunol.,* 1997, vol. 159, 3508-3518
- *Nature,* 1989, vol. 337, 661-663
- *J.Bio.Chem,* 1993, vol. 268, 9526-9532
- *J.Exp.Med.,* 1985, vol. 162, 2163-2168
- *Brit.J.Pharmaco,* 2002, vol. 135, 855-875
- *Drug Discov.Today,* 1997, vol. 2, 273-282
- *Annu.Rev.Immunol,* 1996, vol. 14, 397-440
- *Annu.Rep.Med.Chem,* 1997, vol. 32, 241-250
- *Lancet,* 1999, vol. 354, 1932-1939
- *Gastroenterology,* 1995, vol. 109, 129-135
- *N.Engl.J.Med,* 1999, vol. 340, 1398-1405
- *J.Pharm.Pharmacol,* 2003, vol. 55 (8), 1107-1114
- *J.Invest.Dermatol,* 1996, vol. 107, 51-56
- *Curr.Pharma.Design,* 2002, vol. 8, 1255-1296
- *Annu.Rev.Med,* 1994, vol. 45, 491-503
- *Curr.Opin.Cell Biol,* 2001, vol. 5, 432-438
- *Trends Pharmacol.Sci,* 1997, vol. 18 (5), 164-71
- *Br.J.Pharmacol,* 1995, vol. 115, 39-46
- *Nat.Rev.Drug Discov,* 2003, vol. 2, 736-746
- *Arthritis.Res,* 2002, vol. 4, 17-524
- *Drug News Perspect,* 1996, vol. 9, 261-269
- *J.Inflamm,* 1996, vol. 46, 86-97
- *J.Med.Chem.,* 2001, vol. 44 (16), 2511-2522
- *J.Med.Chem.,* 2001, vol. 44 (16), 2523-2535
- *J.Med.Chem.,* 2002, vol. 45 (12), 2526-2533
- *Chem.Ber,* 1990, vol. 123, 523-533
- *Chem.Ber,* 1990, vol. 123, 1885-1889
- *Tetrahedron Lett.,* vol. 41 (32), 6241-6244
- *J.Org.Chem.,* 1998, vol. 63 (17), 6023-6026
- *J.Org.Chem.,* 2001, vol. 66 (23), 7729-7737
- *J.Org.Chem.,* 2000, vol. 65 (4), 1144-1157
- *Org.Lett,* 2003, vol. 5 (14), 2453-2456
- *Eur.J.Org.Chem.,* 1999, vol. 9, 2373-2381
- *Igakunoayumi,* 2004, vol. 210 (10), 883-887
- *Org.Lett,* 2002, vol. 4 (4), 581-584
- *JPET,* 1998, vol. 287, 705-711
- *Eur.J.Pharmacol.,* 1995, vol. 286, 281-290
- *Curr.Ther.Res,* 1985, vol. 38, 23-29
- *Lancet,* 2001, vol. 358, 265-270
- *Expert Opinion on Investigational Drugs,* 2001, vol. 10 (4), 1361-1379

- *Neuropsychopharmacology,* 2000, vol. 22, 42-51
- *Prog.Nucleic Acid Res.Mol.Biol,* 2001, vol. 69, 249-315
- *J.Neurosci.,* 1999, vol. 19, 610-618
- *Eur.J.Pharmaco,* 2004, vol. 498, 135-142
- *Biochemica et Biophysica acta,* 1984, vol. 797, 354-362
- *Neuropsychopharmacology,* 1983, vol. 22, 267-272
- *Bioorg.Med.Chem.Lett,* 2004, vol. 14 (21), 5275-5280
- *J.Org.Chem.,* 2004, vol. 1, 129-137
- *J. Pharmacol. Exp. Ther,* 2000, vol. 302, 390-396
- *J. Invest. Dermatol,* 1995, vol. 105, 749-755
- *J. Immuno,* 1997, vol. 159, 2484-2491
- *Pharmacol,* 2005, vol. 75, 45-52
- *Br. J. Dermatolol,* 2004, vol. 151, 1133-1142